# EUROPEAN PATENT SPECIFICATION

(11) **EP 1 071 805 B1**
(45) Date of publication and mention of the grant of the patent: **27.07.2011**
(21) Application number: 99918712.3
(22) Date of filing: 19.04.1999
(51) Int. Cl.: C12N 15/86, C12N 15/28, C12N 15/55, A61K 48/00

(54) **ADENOVIRAL VECTORS FOR TREATING DISEASE**
ADENOVIRALE VEKTOREN ZUR BEHANDLUNG DER ERKRANKUNGEN
VECTEURS ADENOVIRAUX DESTINES AU TRAITEMENT DE MALADIES

(30) Priority: 24.04.1998 US 83033 P; 25.01.1999 US 117103 P
(43) Date of publication of application: 31.01.2001
(73) Proprietor: ONYX PHARMACEUTICALS, INC., Emeryville, CA 94608 (US)
(72) Inventor: HERMISTON, Terry, Corte Madera, CA 94925 (US); HAWKINS, Lynda, K., Richmond, CA 94806 (US); JOHNSON, Leisa, Lafayette, CA 94549 (US)
(74) Representative: Cripps, Joanna Elizabeth
(86) International application number: PCT/US1999/008656
(87) International publication number: WO 1999/055831

(56) References cited:
- WO-A-95/25807
- BETT ET AL: "DNA sequence of the deletion/insertion in early region 3 of Ad5 dl309" VIRUS RESEARCH,NL,AMSTERDAM, vol. 39, no. 1, page 75-82-82 XP002106221 ISSN: 0168-1702
- DATABASE GENBANK [Online] ACCESSION NUMBER M73260; REL. 28, 18 July 1991 (1991-07-18) "MASTADENOVIRUS H5 GENE, COMPLETE GENOME" XP002126555
- TUBOLY ET AL: "RESTRICTION ENDONUCLEASE ANALYSIS AND PHYSICAL MAPPING OF THE GENOME OF PORCINE ADENOVIRUS TYPE 5" VIRUS RESEARCH, vol. 37, 1995, pages 49-54, XP002126550
- GRAHAM F L ET AL: "METHODS FOR CONSTRUCTION OF ADENOVIRUS VECTORS" MOLECULAR BIOTECHNOLOGY,US,TOTOWA, NJ, vol. 3, no. 3, page 207-220 XP000603535 ISSN: 1073-6085
- CHROBOCZEK ET AL: "THE SEQUENCE OF THE GENOME OF ADENOVIRUS TYPE 5 AND ITS COMPARISON WITH THE GENOME OF ADENOVIRUS TYPE 2" VIROLOGY, vol. 186, 1992, pages 280-285, XP002126551
- SAMBROOK ET AL: "MOLECULAR CLONING. A LABORATORY MANUAL. SECOND EDITION" 1989 , COLD SPRING HARBOR LABORATORY PRESS XP002126554 page 5.3 -page 5.9
- SPARER ET AL: "THE ROLE OF HUMAN ADENOVIRUS EARLY REGION 3 PROTEINS (GP19K, 10.4K, 14.5K, AND 14.7K) IN A MURINE PNEUMONIA MODEL" JOURNAL OF VIROLOGY, vol. 70, 1996, pages 2431-2439, XP002126552
- BETT ET AL: "AN EFFICIENT AND FLEXIBLE SYSTEM FOR CONSTRUCTION OF ADENOVIRUS VECTORS WITH INSERTIONS OR DELETIONS IN EARLY REGIONS 1 AND 3" PROCEEDINGS OF THE NATIONAL ACADEMY OF SCIENCES, USA, vol. 91, 1994, pages 8802-8806, XP002126553 cited in the application
- HU S -X ET AL: "DEVELOPMENT OF AN ADENOVIRUS VECTOR WITH TETRACYCLINE-REGULATABLE HUMAN TUMOR NECROSIS FACTOR ALPHA GENE EXPRESSION" CANCER RESEARCH,US,AMERICAN ASSOCIATION FOR CANCER RESEARCH, BALTIMORE, MD, vol. 57, no. 16, page 3339-3343 XP002068734 ISSN: 0008-5472
- HAJ-AHMAD Y ET AL: "DEVELOPMENT OF A HELPER-INDEPENDENT HUMAN ADENOVIRUS VECTOR AND ITSUSE IN THE TRANSFER OF THE HERPES SIMPLEX VIRUS THYMIDINE KINASE GENE", JOURNAL OF VIROLOGY, THE AMERICAN SOCIETY FOR MICROBIOLOGY, US, vol. 57, no. 1, 1 January 1986 (1986-01-01), pages 267-274, XP002058870, ISSN: 0022-538X
- BETT A J ET AL: "PACKAGING CAPACITY AND STABILITY OF HUMAN ADENOVIRUS TYPE 5 VECTORS", JOURNAL OF VIROLOGY, THE AMERICAN SOCIETY FOR MICROBIOLOGY, US, vol. 67, no. 10, 1 October 1993 (1993-10-01), pages 5911-5921, XP002921148, ISSN: 0022-538X
- HAJ-AHMAD Y; GRAHAM F L: "DEVELOPMENT OF A HELPER-INDEPENDENT HUMAN ADENOVIRUS VECTOR AND ITSUSE IN THE TRANSFER OF THE HERPES SIMPLEX VIRUS THYMIDINE KINASE GENE" JOURNAL OF VIROLOGY, THE AMERICAN SOCIETY FOR MICROBIOLOGY, US, vol. 57, no. 1, 1986, pages 267-274, XP002058870
- BETT A J; ET AL: "PACKAGING CAPACITY AND STABILITY OF HUMAN ADENOVIRUS TYPE 5 VECTORS" JOURNAL OF VIROLOGY, NEW YORK, US, US, vol. 67, no. 10, October 1993 (1993-10), pages 5911-5921, XP002921148

## Description

### Field of the Invention

The invention described herein relates generally to the field of gene therapy, and more specifically to adenoviral vectors that have prophylactic or therapeutic applications.

### Background of the Invention

Adenovirus is a vector of choice for performing gene therapy. See, Jolly, D., Cancer Gene Therapy, vol. 1, no. 1. 1994: pp51-64. The well-characterized molecular genetics of adenovirus render it an advantageous vector in this regard. Adenoviruses are nonenveloped icosohedral double-stranded DNA viruses with a linear genome of approximately 36 kilobase pairs. Each end of the viral genome has a short sequence known as the inverted terminal repeat (or ITR), which is required for viral replication. Portions of the viral genome can be readily substituted with DNA of foreign origin, and furthermore, recombinant adenoviruses are structurally stable.

The adenovirus replication cycle has two phases: and early phase, during which 4 transcription units E1, E2, E3, and E4 are expressed, and a late phase which occurs after the onset of viral DNA synthesis when late transcripts are expressed from the major late promoter (MLP). The late messages encode most of the viruses structural proteins. The gene products of E1, E2 and E4 are responsible for transcriptional activation, cell transformation, viral DNA replication, as well as other viral functions, and are necessary for viral growth.

To date most adenoviral vectors are based on viruses mutated in E1, E3 or a site upstream of E4 which provide for sites for the insertion of foreign DNA. Perhaps the majority of vectors are based on mutant strains of adenovirus which lack the E 1 region of the genome. By deleting this region, the virus is rendered replication incompetent, and additionally, a region is made available for the insertion of foreign genes.

There are numerous reports on the use of adenovirus for gene therapy. For example, Smith, et al., Nature Genetics, Vol. 5, pgs. 397-402 (1993) discloses the administration to mice of an adenoviral vector including a human Factor IX gene. Such administration resulted in efficient liver transduction and plasma levels of human Factor IX that would be therapeutic for hemophilia B patients. Human Factor IX levels, however, slowly declined to baseline by nine weeks after injection, and were not reestablished by a second vector injection. Smith, et al., also found that neutralizing antibodies to adenovirus block successful repeat administration of the adenovirus.

Kozarsky, et al., J. Biol. Chem., Vol. 269, No. 18. pgs. 13695-13702 (May 6, 1994) discloses the infusion of an adenoviral vector including DNA encoding the LDL receptor to rabbits. Stable expression of the LDL receptor gene was found in the rabbits for 7 to 10 days, and diminished to undetectable levels within 3 weeks. The development of neutralizing antibodies to the adenovirus resulted in a second dose being completely ineffective.

Kass-Eisler, et al., Gene Therapy, Vol. 1, pgs. 395-402 (1994) suggest that a T-cell response contributes to, but is not responsible solely for, the limited duration of expression in adults from adenovirus vectors. The authors further show that cyclosporin A is not effective in blocking the humoral response to the vector.

Fang, et al.. J. Cell. Biochem., Supplement 21A, C6-109. pg 363 (1995) disclose the attempted re-injection of an adenovirus vector in dogs which were treated with cyclosporin A, an immunosuppressive agent. Such attempted re-injection was unsuccessful.

Yang, et al.. Proc. Nat. Acad. Sci., Vol. 91. pgs. 4407-4411 (May 1994) describe recombinant adenoviruses in which the E1a and E1b regions have been deleted. Such viruses also include a transgene. When these adenoviruses are administered to an animal host, cells harboring the recombinant viral genome express the transgene as desired: however, low level expression of viral genes also occurs.

As exemplified above, adenoviruses can be efficient in gene transfer into cells in vivo, and thus may be employed as delivery vehicles for introducing desired genes into eukaryotic cells, whereby the adenovirus delivers such genes to eukaryotic cells by binding cellular receptors. There are, however, several limitations to adenovirus gene transfer which are due in part to host responses directed at either the adenovirus vector particle, breakdown products of the vector particle, or the transduced cells. These host responses include non-specific responses and specific immune responses. The non-specific responses include inflammatory and non-inflammatory changes. An example of the latter is a change in host cell gene expression. Specific immune responses include various cellular responses and humoral antibody responses. Cellular responses include those mediated by T-helper lymphocytes, T-suppressor lymphocytes, cytotoxic T lymphocytes (CTL), and natural killer cells.

Despite the high efficiency of adenovirus vector mediated gene transfer, the transient nature of adenovirus vector mediated gene transfer has suggested that repeat administrations of adenovirus vectors may be necessary. Recent studies in cotton rats, however, have demonstrated that host immune responses directed towards adenoviral vectors correlate with decreased efficiency of gene transfer and expression after repeated administration. Yei et al.. Gene Therapy, 1:192-200 (1994). The E3 region encodes several immunoregulatory proteins, which are not required for viral replication; gp 19K, 10.4K, 14.5K and 14.7, and one protein, 11.6K, that is required for lysis of infected cells, and release of infectious progeny.

While the E3 region is not essential for viral replication, it does play a key role in modulating the host immune response to the virus. For instance, it is known that gp19K binds to MHC class 1 molecules in the endoplasmic reticulum, thus inhibiting its glycoslation and transport to the surface of the virally infected cells Consequently, the infected cells are not recognized as foreign by cytotoxic lymphocytes Sec. Burgert. B. et al. Proc Natl Acad Sci USA 1987, vol 8 1356-60

Because of the many functions of the E3 region. It would be desirable to have an adenoviral vector for gene therapy applications which would permit one to delete particular regions of E3. and substitute foreign DNA, depending on the intended application of the vector. For example, there is described deletions in the E3 region that result in the removal of 1.88 kb, between the Xba I sites Sec. Berkner, K and Sharp. P.. (1983) Nucleic Acids Res Vol 11, pages 6003-6020. and Haj-Ahmad. Y and Graham. F (1936) J Virol Vol 57. pages 267-27.1 Further there is described compositions and methods for constructing adenovirus having insertions or deletions in both the E1 and E3 regions See also. Ginsberg. H S et al . Proc Natl Acad Sci USA 1989, vol 86, pp 3823-7

Thus, although there exists vectors having mutations in the E3 region, or large parts of the region deleted, to date there does not exist a vector that allows one to remote select parts of the region and substitute foreign DNA

### Summary of the Invention

A first object of the invention is to describe recombinant viral vectors that have restriction sites engineered into the E3 region that facilitate partial or total deletion of this region, or select genes contained therein, and if desired, substituted therefor a heterologous gene, which gene will exhibit an expression pattern, both in terms of time and degree of expression, similar to the endogenous adenoviral gene it replaces.

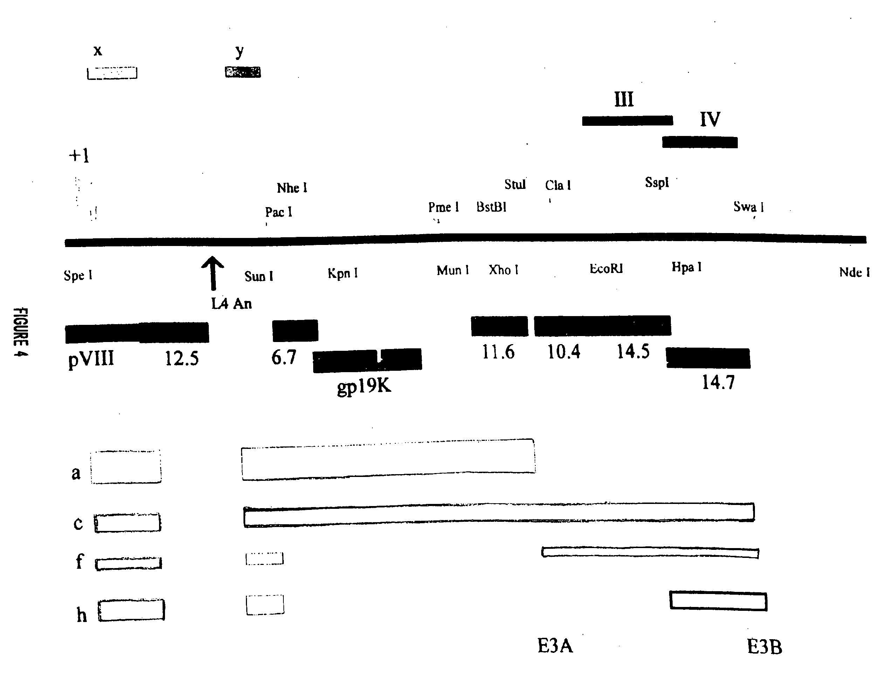

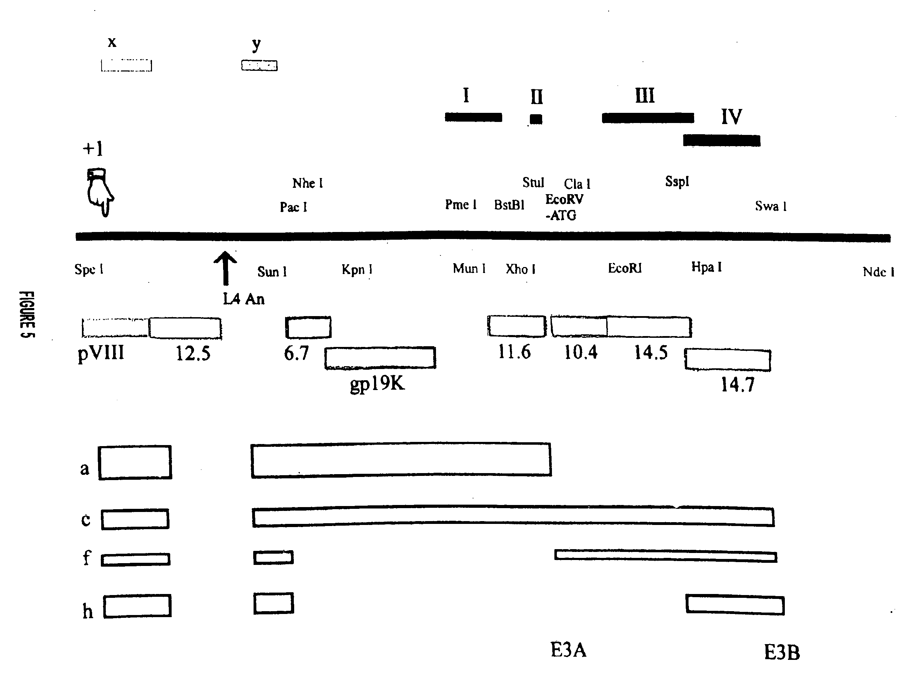

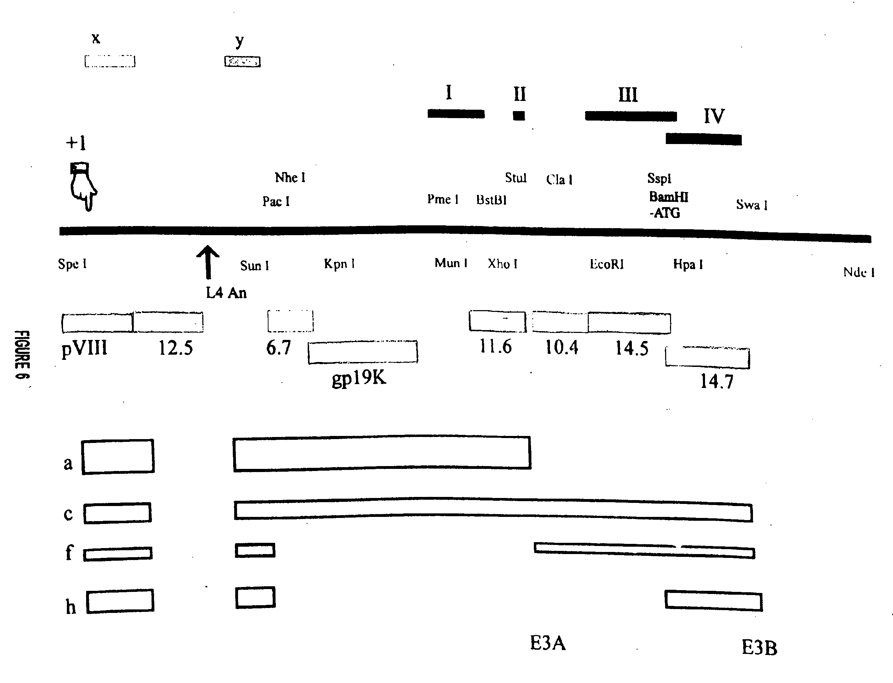

Accordingly, there is provided a recombinant adenoviral vector comprising restriction sites in the E3 region of said vector selected from the group consisting of Pac1, Cla1, Pme1, Swa1, BamH1, BstB1, Ssp1, Nhe1, Stu1 and EcoR1,
wherein said restriction sites have relative positions as shown in Figures 4 to 7;
wherein restriction sites are introduced by way of mutation preferably without changing the coding sequence, but where amino acid changes are made they are conservative in nature; and
wherein restriction sites are positioned so as not to disrupt critical splicing and polyadenylation signals;
wherein restriction sites facilitate partial or total deletion of an adenoviral gene or genes contained in said E3 region, and substitution of said partial or total deletion of said adenoviral gene or genes with a heterologous gene,
wherein said heterologous gene exhibits an expression pattern, both in terms of timing and degree of expression, similar to the endogenous adenoviral gene it replaces;
wherein said vector includes restriction sites in the early region genes of the E3 region that encode the 6.7k and gp19k proteins and/or
wherein said vector includes restriction sites in the early region genes of the E3 region that encode the 10.4k, 14.5k and the 14.7k proteins.

A second object of the present invention is to describe method for making recombinant adenoviral vectors that have restrictions sites in the E3 region and that facilitate partial or total deletion of the E3 region or select genes contained therein. Accordingly, there is provided a method of producing a recombinant adenoviral vector for use in expressing a heterologous gene, said method comprising engineering a restriction site into the E3 region of said vector selected from the group consisting of Pac1, Cla1, Pme1, Swa1, BamH1, BstB1, Ssp1, Nhe1, Stu1 and EcoRV;
wherein said restriction sites have relative positions as shown in Figures 4 to 7;
which restriction site enables the partial or total deletion of an adenoviral gene or genes contained in said E3 region, and substitution of said partial or total deletion of said adenoviral gene or genes with a heterologous gene; said restriction site being positioned such that said heterologous gene exhibits an expression pattern, both in terms of timing and degree of expression, similar to the endogenous adenoviral gene it replaces without disrupting critical splicing and polyadenylation signals;
wherein any amino acid changes are conservative in nature;
wherein said restriction site is engineered into the early region genes of the E3 region that encode the 6.7k and gp19k proteins; and/or
wherein said restriction site is engineered into the early region genes of the E3 region that encode the 10.4k, 14.5k and the 14.7k proteins.

Also described are recombinant adenoviral mutants that have restriction sites in the E3 region that facilitate partial or total deletion of the E3 region or select genes contained therein.

Further described are recombinant adenoviral mutants that have restriction sites in the E3 region that facilitate partial or total deletion of the E3 region, or select genes contained therein, including virions: E3SV. E3SV +V, E3SV + B, and E3SV +V +B

A ninth object of the invention is a description of recombinant adenoviral vectors of the invention that have restriction sites in the E3 region that facilitate partial or total deletion of the E3 region wherein such vectors also have mutations elsewhere in the adenoviral genome, preferably in the EIA. EIB, and/or E4 regions.

A tenth object of the invention is a description of the use of recombinant adenoviral vectors that have restriction sites in the E3 region that facilitate partial or total deletion of the E3 region, or select genes contained therein, therein the adenoviral mutants have substituted in the E3 region genes that encode medically beneficial proteins for the preparation of a medicament for treating cancer in a mammal. Preferred substituted genes include heterologous genes including negative selection genes, preferably cytosine deaminase, and thymidine kinase.

These and other objects of the present invention will become apparent to one of ordinary skill in the art upon reading the description of the various aspects of the invention in the following specification The foregoing and other aspects of the present invention are explained in greater detail in the drawings, detailed description, and examples set forth below.

### Brief Description of the Drawings

Figure 1 shows a map of the E3 region transcriptional unit of adenovirus type 5. The split arrows indicate the spliced structures of the mRNAs (open rectangles or solid lines represent Exons: dashed lines. Introns): the thickness of the arrow indicates the relative abundance. The shaded bars above the arrows indicate the E3 proteins, which arc named on the basis of their molecular masses
Figure 2 shows the production of recombinant virus using pNB and Ad5 TP-DNA (m. u. stands for map units).
Figure 3 shows the production of recombinant virus using pSN and Ad5 TP-DNA
Figure 4 Shows the restriction map of the E3 region of the adenovirus E3SV
Figure 5 shows the restriction map of the E3 region of the adenovirus E3SV +V
Figure 6 shows the restriction map of the E3 region of the adenovirus E3SV +B
Figure 7 shows the rcstncuon map of the E3 region of the adenovirus E3SV + V+B
Figure 8 shows A549 cells mock infected or infected with Ad5
Figure 9 sho%%.s A549 cells infected with viruses 301, 302, 303, and 304
Figure 10 shows western blot analysis of gp19k from cell lysates prepared from cells infected with viruses 301, 302, 303, and 304 at different times post infection.
Figure 11 shows a CD assay on cell lysates prepared from cells infected with viruses 301, 302, 303, 304, and 305 at different times post infection.
Figure 12. shows a CD assay on cell lysates prepared from cells infected with viruses 301, 302. 303. 304. and 305 at different times post infection using 0.6 ug protein/reaction.

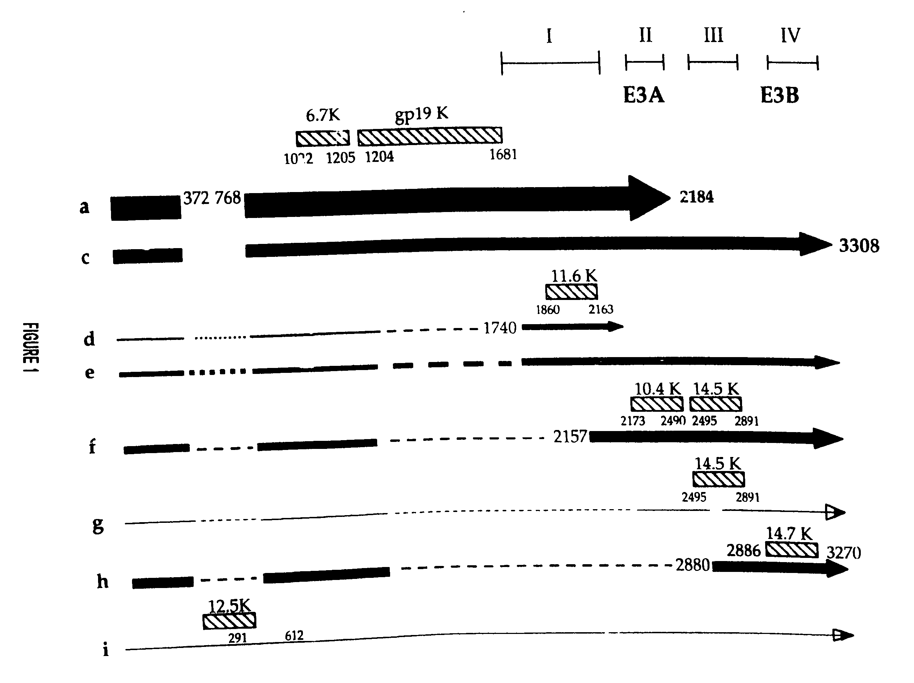

Figure 1 shows the cytopathic effect of viruses 305 and 320 at different times post infection.

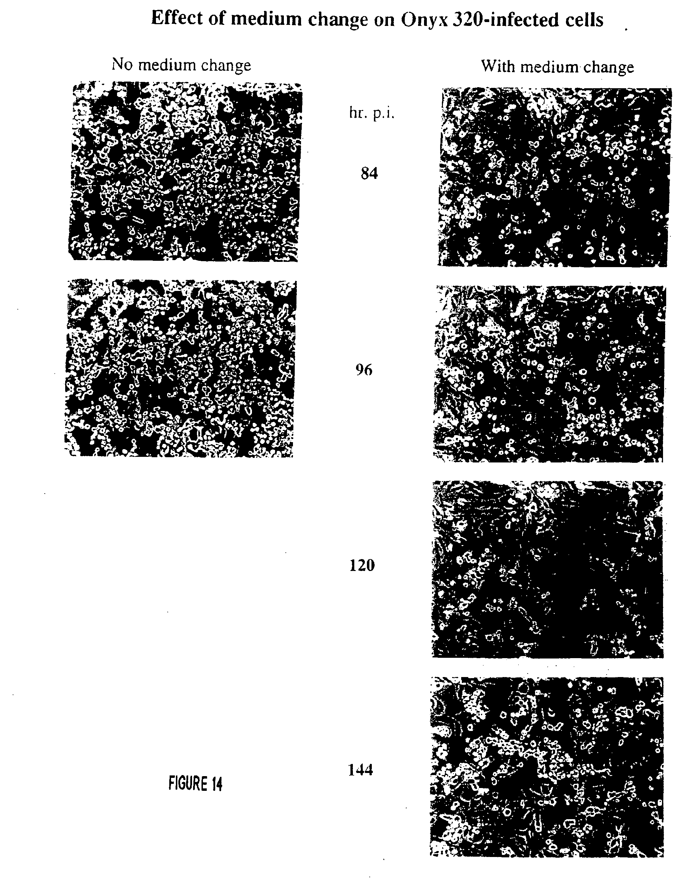

Figure 14 shows the cytopathic effect of virus 320 on cells that have or have not have medium changes at certain times post infection

### Detailed Description of the Invention

### Definitions

Unless defined otherwise, all technical and scientific terms used herein have the same meaning as commonly understood by one of ordinary skill in the art to which this invention belongs. Generally, the nomenclature used herein and the laboratory procedures described below are those well known and commonly employed in the art.

Standard techniques are used for recombinant nucleic acid methods, polynucleotide synthesis, and microbial Culture and transformation (e.g.. electroporation, lipofection). Generally, enzymatic reactions and purification steps are performed according to the manufacturer's specifications. The techniques and procedures arc generally performed according to conventional methods in the art and various general references (see generally. Sambrook et al., Molecular Cloning: A Laboratory Manual. 2nd, edition (1989) Cold Spring Harbor Laboratory Press. Cold Spring Harbor. N.Y.) which are provided throughout this document. The nomenclature used herein and the laboratory procedure in analytical chemistry, organic synthetic chemistry, and pharmaceutical formulation described below arc those well know and commonly employed in the art. Standard techniques are used for chemical syntheses, chemical analyses, pharmaceutical formulation and delivery, and treatment of patents.

Those skilled in the an will also recognize publications that facilitate genetic engineering of the invention adenovirus to produce mutants in the E3 region. Such would include McGrory, W.. J. et al., (1988) Virology, vol. 177, pp. 437-444 who describe insertion of DNA into the E1 region. Hanke. T. et al, (1990) Virology. vol. 177, pp. 437-444 and Bett. A. J. et al. (1993) J. Virol. vol. 67. pp. 5911-5921 who describe insertion of foreign DNA into the E3 region: and Bett, A. J. et al, (1994) Proc. Natl. Acad. Sci. vol. 91. pages 8802-8806, who describe insertion of DNA into the E1 and E3 regions.

In the formulae representing selected specific embodiments of the present invention, the amino- and carboxy-terminal groups, although often not specifically shown, will be understood to be in the form they would assume at physiological pH values, unless otherwise specified. Thus, the N-terminal H₂⁻ and C-terminal-O⁻ at physiological pH arc understood to be present though not necessarily specified and shown, either in specific examples or in generic formulas. In the polypeptide notation used herein, the lefthand end of the molecule is the amino terminal end and the righthand end is the carboxy-terminal end, in accordance with standard usage and convention. Of course, the basic and acid addition salts including those which are formed at nonphysiological Ph values are also included in the compounds of the invention. The amino acid residues described herein are preferably in the "L" isomeric form. Stereoisomers (e.g., D-amino acids) of the twenty conventional amino acids, unnatural amino acids such as a,a-distributed amino acids, N-alkyl amino acids, lactic acid, and other unconventional amino acids may also be suitable components for polypeptides of the present invention, as long as the desired functional property is retained by the polypeptide. For the peptides shown, each encoded residue where appropriate is represented by a three letter designation, corresponding to the trivial name of the conventional amino acid, in keeping with standard polypeptide nomenclature (described in J. Biol. Chem., 243:3552-59 (1969) and adopted at 37 CFR §1.822(b)(2)). Free functional groups, including those at the carboxy- or amino-terminus, referred to as noninterfering substituents, can also be modified by amidation, acylation or other substitution, which can, for example, change the solubility of the compounds without affecting their activity.

As employed throughout the disclosure, the following terms, unless otherwise indicated, shall be understood to have the following meanings:

The term "isolated protein" referred to herein means a protein of cDNA, recombinant RNA, or synthetic origin or some combination thereof, which by virtue of its origin the "isolated protein" (1) is not associated with proteins found in nature, (2) is free of other proteins from the same source, e.g. free of human proteins, (3) is expressed by a cell from a different species, or (4) does not occur in nature.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "adenovirus" as referred to herein indicates over 40 adenoviral subtypes isolated from humans, and as many from other mammals and birds. See, Strauss, "Adenovirus infections in humans," in The Adenoviruses, Ginsberg, ed., Plenum Press, New York, NY, pp. 451-596 (1984). The term preferably applies to two human serotypes, Ad2 and Ad5.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "oligonucleotide" referred to herein includes naturally occurring, and modified nucleotides linked together by naturally occurring, and non-naturally occurring oligonucleotide linkages. Oligonucleotides are a polynucleotide subset with 200 bases or fewer in length. Preferably oligonucleotides are 10 to 60 bases in length. Oligonucleotides are usually single stranded, e.g. for probes: although oligonucleotides may be double stranded, e.g. for use in the construction of a gene mutant. Oligonucleotides of the invention can be either sense or antisense oligonucleotides. The term "naturally occurring nucleotides" referred to herein includes deoxyribonucleotides and ribonucleotides. The term "modified nucleotides" referred to herein includes nucleotides with modified or substituted sugar groups and the like known in the art.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Various methods of labeling polypeptides and glycoproteins are known in the art and may be used. Examples of labels for polypeptides include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), enzymatic labels (e.g.. horseradish peroxidase, b-galactosidase, luciferase, alkaline phosphatase), chemiluminescent, biotinyl groups, predetermined polypeptide epitopes recognized by a secondary reporter (e.g.. leucine zipper pair sequences, binding sites for secondary antibodies, metal binding domains, epitope tags). In some embodiments, labels are attached by spacer arms of various lengths to reduce potential steric hindrance.

The term "sequence homology" referred to herein describes the proportion of base matches between two nucleic acid sequences or the proportion amino acid matches between two amino acid sequences. When sequence homology is expressed as a percentage, e.g., 50%, the percentage denotes the proportion of matches over the length of sequence that is compared to some other sequence. Gaps (in either of the two sequences) are permitted to maximize matching; gap lengths of 15 bases or less are usually used, 6 bases or less are preferred with 2 bases or less more preferred.

The term "selectively hybridize" referred to herein means to detectably and specifically bind. Polynucleotides, oligonucleotides and fragments of the invention selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the invention and a nucleic acid sequence of interest will be at least 80%, and more typically with preferably increasing homologies of at least 85%, 90%, 95%, 99%, and 100%.

Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. See Dayhoff, M.O., in Atlas of Protein Sequence and Structure, 1972, volume 5, National Biomedical Research Foundation, pp. 101-110, and Supplement 2 to this volume, pp. 1-10. The two sequences or parts thereof are more preferably homologous if their amino acids are greater than or equal to 50% identical when optimally aligned using the ALIGN program.

The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotides: "reference sequence", "comparison window". "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing may comprise a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window," as may be used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection, and the best alignment (i.e., resulting in the highest percentage of homology over the comparison window) generated by the various methods is selected. The term "sequence identity," means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and lustidine; and a group of amino acids having suffur-containing side chains is cysteine and methionne. Preferred conservative amino acids substitution groups are valine-leucine-isoleucine, phenylalanine-tyrosine, Lysine-arginine, alanine-valine, glutamic-aspartic, and asparagine-glutamine.

The term "polypeptide fragment" or "peptide fragment" as used herein refers to a polypeptide that has an amino-terminal and/or carboxy-terminal deletion, but where the remaining amino acid sequence is identical to the corresponding positions in the naturally-occurring sequence deducted, for example, from a full-length cDNA sequence. Fragments typically 8-10 amino acids long, preferably at least 10-20 amino acids long, and even more preferably 20-70 amino acids long.

Other chemistry terms herein are used according to conventional usage in the art, as exemplified by. The McGraw-Hill Dictionary of Chemical Terms (ed. Parker. S., 1985). McGraw-Hill. San Francisco.

The production of proteins from cloned genes by genetic engineering is well known. See e.g U.S. Patent Number 4.761.371 to Bett et al, at column 6. line 3 to column 9- line 65. The discussion which follows is accordingly intended as an overview of this field, and is not intended to reflect the full state of the art.

DNA which encodes proteins that may be inserted into the adenoviral constructs of the instant invention in the E3 region can be obtained, in view of the instant disclosure by chemical synthesis by screening reverse transcripts of mRNA from appropriate cells or cell line cultures, by screening genomic librancs from appropriate cells, or by combinations of these procedures, as illustrated below. Screening of mRNA or genomic DNA may be carried out with oligonucleotide probes generated from known gene sequence information. Probes may be labeled with a detectable group such as a fluorescent group, a radioactive atom or a chemiluminescent group in accordance with known procedures and used in conventional hybridization assays, as described in greater detail in the Examples below.

In the alternative, a gene sequence may be recovered by use of the polymerase chain reaction (PCR) procedure. See U.S. Patent Numbers 4,683,195 to Mullis et al. and 4,683,202 to Mullis.

A vector is a replicable DNA construct. Preferred embodiment vectors described herein to realize the adenovirus E3 mutants are based on the pGEM vector series of Promega Corporation. Vectors are used either to amplify DNA encoding a desired protein and/or to express DNA which encodes the protein. An expression vector is a replicable DNA construct in which a DNA sequence encoding a protein of interest is operably linked to suitable control sequences capable of effecting the expression of the protein in a suitable host. The need for such control sequences will vary depending upon the host selected and the transformation method chosen. Generally control sequences include a transcriptional promoter an optional operator sequence to control transcription, a sequence encoding suitable mRNA ribosomal binding sites, and sequences which control the termination of transcription and translation. Amplification vectors do not require expression control domains. All that is needed is the ability to replicate in a host usually conferred by an origin of replication, and a selection gene to facilitate recognition of transformants.

Vectors useful for practicing the present invention include plasmids, viruses (including phage), and integratable DNA fragments (i.e.. fragments integratable into the host genome by homologous recombination). The vector replicates and functions independently of the host genome or may, in some instances, integrate into the genome itself. Suitable vectors will contain replicon and control sequences which are derived from species compatible with the intended expression host. Transformed host cells are cells which have been transformed or transfected with the vectors constructed using recombinant DNA techniques.

DNA regions are operably linked when they are functionally related to each other. For example, a promoter is operably linked to a coding sequence if it controls the transcription of the sequence; a ribosome binding site is operably linked to a coding sequence if it is positioned so as to permit translation. Generally, operably linked means contiguous and in the case of leader sequences, contiguous and in reading frame. A preferred promoter in those instances where certain E3 region DNA is deleted and DNA substitued therein is a tissue specific promoter which is operably linked to a negative selection gene.

Suitable host cells include prokaryotes, yeast cells, or higher eukaryotic cells. Prokaryotes include gram negative or gram positive organisms, for example *Escherichia coli* (*E. coli*) or Bacilli Higher eukaryotic cells include established cell lines of mammalian origin as described below. Exemplary host cells are DH5a. *E. coli* W3110 (ATCC 27,325). *E. coli* B. *E. coli* X1776 (ATCC 31,537) and *E. coli* 294 (ATCC 31,446).

A broad variety of suitable microbial vectors are available, and may have applications in constructing the instant adenoviral vectors. Generally, a microbial vector will contain an origin of replication recognized by the intended host, a promoter which will function in the host and a phenotypic selection gene such as a gene encoding proteins conferring antibiotic resistance or supplying an autotrophic requirement. Similar constructs will be manufactured for other hosts. *E. coli* is typically transformed using pBR322. See Bolivar et al. Gene 2, 95 (1977). pBR322 contains genes for ampicillin and tetracycline resistance and thus provides easy means for identifying transformed cells. Expression vectors should contain a promoter which is recognized by the host organism. Thus generally means a promoter obtained from the intended host. Promoters most commonly used in recombinant microbial expression vectors include the beta-lactamase (penicillinase) and lactose promoter systems (Chang et al., Nature 275, 615 (1978): and Goeddel et al., Nucleic Acids Res 8,4057 (1980) and EPO Application Publication Number 36,776) and the *tac* promoter (H. De Boer et al., Proc. Natl. Acad. Sci. USA 80, 21 (1983)). While these are commonly used, other microbial promoters are suitable. Details concerning nucleotide sequences of many promoters have been published, enabling a skilled worker to operably ligate them to DNA in plasmid or viral vectors (Siebenlist et al., Cell 20. 269. 1980)).

Cultures of cells derived from multicellular organisms are a desirable host for recombinant protein synthesis. In principal, any higher eukaryotic cell culture is workable, whether from vertebrate or invertebrate culture. However, mammalian cells are preferred. Propagation of such cells in cell culture has become a routine procedure. See Tissue Culture, Academic Press, Kruse and Paterson, editors (1973). Examples of useful host cell lines are VERO and HeLa cells, Chinese hamster ovary (CHO) cell lines, and FL5.12, WI138, BHK, COS-7, CV, and MDCK cell lines. Expression vectors for such cells ordinarily include (if necessary) an origin of replication, a promoter located upstream from the gene to be expressed, along with a ribosome binding site, RNA splice site (if intron-containing genomic DNA is used), a polyadenylation site, and a transcriptional termination sequence.

An origin of replication may be provided either by construction of the vector to include an exogenous origin, such as may be derived from SV40 or other viral source (e.g. Polyoma. Adenovirus, VSV. or BPV), or may be provided by the host cell chromosomal replication mechanism. If the vector is integrated into the host cell chromosome, the latter may be sufficient.

The transcriptional and translational control sequences in expression vectors to be used in transforming vertebrate cells are often provided by viral sources, including adenovirus. A variety of viral and mammalian constitutive promoter elements can be used. See, Mittal et al., (1993) Virus Research, vol. 28, pp. 67-90. For example, commonly used promoters are derived from polyoma. Adenovirus 2, and Simian Virus 40 (SV40). See, e.g., U.S. Patent Number 4,599,308. The early and late promoters are useful because both are obtained easily from the virus as a fragment which also contains the SV40 viral origin of replication. See Fiers et al., Nature 273, 113 (1978).

### Construction of Adenovirus E3 Mutants

Methods for the construction of adenoviral mutants are generally known in the art. See, Mittal, S. K., Virus Res., 1993, vol: 28, pages 67-90. Further, the adenovirus 5 genome is registered as Genbank accession #M73260, and the virus is available from the American Type Culture Collection, Rockville, Maryland, U. S. A., under accession number VR-5.

Generally, adenovirus vector construction involves an initial deletion or modification of a desired region of the adenoviral genome, preferably the Ad5 genome, in a plasmid cassette using standard techniques. For example, the NdeI fragment of Ad5, corresponding to bases 19549-31089, can be excised from the Ad5 genome and inserted into an appropriate plasmid, preferrably pGEM5zf+, available from Promega Corporation. This plasmid is termed pNB, and is described more in detail in the Example section, below.

The adenoviral DNA, or a fragment thereof present in pNB and which corresponds to the E3 region of the virus is subsequently cloned into another plasmid which may also be pGEM5zf+. For example, the SpeI-NdeI fragment, corresponding to bases 27082-31089, of the Ad5 genome can be excised from pNB and cloned into the SpeI and NdeI sites in the multiple cloning site (MCS) of pGEM5zf+. This vector is termed pSN, and the adeno DNA present therein, bases 27082-31089, may be used to engineer the desired restriction sites into the E3 region to yield the appropriate E3 vectors, plasmids and viruses, discussed more below.

Certain of the materials and methods used to construct adenovirus mutants are described by Hanke. T., et. al. (1990) Virology. vol. 177. pages 437-444, and Bett, A. J., et. al., (993) J. Virol. vol 67, pages 5911-5921, and in PCT/CA96/00375. Microbix Biosystems. Inc.. located at 341 Bering Avenue. Toronto, Ontario Canada, sells many of the materials used to construct adenovirus mutants, and provides Product Information Sheets on how to make them.

It is noteworthy that while the instant invention is described in terms of adenovirus type 5, it may be practiced with other similar adenovirus serotypes. The general organization of the adenoviral genome is conserved among serotypes, and specific functions are similarly situated.

The mutations in the E3 region described herein may be incorporated into adenoviral mutants that have mutations outside the E3 region. Preferably such mutations would be in the EIb and/or EIa and/or the E4orf6 regions of the adenoviral genome. In the case of EIb mutations, the mutations may confer on adenovirus the ability to preferentially replicate in neoplastic cells compared to normal cells, wherein the neoplastic cells are functionally defective in the tumor suppressor, p53. Such mutations typically occur in the E1B region that encodes the 55k protein. Defective p53 can arise in numerous ways, including a defect in those proteins that interact with p53: that is, a defect in the p53 pathway that renders p53 functionally inactive. See. U. S. Patent No. 5, 677, 178. Thus, the E3 mutations described herein could be combined with the EIB deletion in the adenovirus dl1520. This virus is described by Barker and Berk (1987) Virology 156: 107.

In the case of E1A mutations, the mutations may confer on adenovirus the ability to preferentially replicate in neoplastic cells compared to normal cells, wherein the neoplastic cells are functionally defective in the retinoblastoma tumor suppressor gene product, or p 105 Rb. Such inactivating mutations typically occur in the E1a CR1 domain (amino acids 30-85 in Ad5: nucleotide positions 697-790) and/or the CR2 domain (amino acids 120-139 in Ad5: nucleotide positions 920-967). which are involved in binding the p105 *RB* protein. The CR3 domain of the adenoviral genome (spanning amino acids 150-186) may remain and be expressed as a truncated p289R polypeptide and be functional in transactivation of adenoviral early genes. Defective Rb can arise in numerous ways, including a defect in those proteins that interact with Rb; that is, a defect in the Rb pathway that renders Rb functionally inactive. See, U. S. Patent No. 5, 677, 178. Thus, the E3 mutations described herein could be combined with the E1A deletion in the adenovirus Ad5 NT dl 1010.

Another aspect of the instant invention is the incorporation of heterologous genes into the E1B, E1A, or E4orf6 regions of an E3 mutant virus described herein. Thus, such viruses would contain heterologous genes in E3, and optionally in E1B, E1A or E4orf6. Examples of such heterologous genes, or fragments thereof that encode biologically active peptides, include those that encode immunomodulatory proteins, and prodrug activators (i.e. cytosine deaminase, thymidine kinase, U. S. Patent Nos. 5, 358, 866, and 5, 677, 178). Examples of the former would include interleukin 2, U.S. Patent Nos. 4,738, 927 or 5, 641, 665; interleukin 7, U. S. Patent Nos. 4, 965, 195 or 5, 328, 988; and interleukin 12, U. S. Patent No. 5,457, 038: tumor necrosis factor alpha, U. S. Patent Nos. 4, 677, 063 or 5, 773, 582; interferon gamma, U.S. Patent Nos. 4, 727, 138 or 4, 762, 791: or GM-CSF, U.S. Patent Nos. 5, 393, 870 or 5,391,485. Additional immunomodulatory proteins further include macrophage inflammatory proteins, including MIP-3, (See, Well, T. N. and Peitsch, MC. J. Leukoc. Biol vol 61 (5): pages 545-50,1997), and cell suicide, or apoptosis inducing proteins, including BAD and BAX. See, Yang, E., et al. Cell, vol 80, pages 285-291 (1995): and Sandeep, R., et al Cell, vol. 91, pages 231-241 (1997).

As mentioned above, the initial step in the construction of recombinant adenoviral vectors having novel restriction sites in the E3 region that facilitate partial or total deletion of the E3 region, or select genes contained therein, is to make mutations in the adenoviral genome in a plasmid cassette using well established techniques of molecular biology, referred to herein. The following restrictions sites were engineered into the E3 region of adenovirus 5: PacI, ClaI, PmeI, SwaI. BamHI, BstBI, Sspl, Nhel, and StuI and EcoRV. Their relative positions in the E3 region are show in figures 4-7. The restriction sites were positioned so as not to disrupt critical splicing and polyadenylation signals. Another consideration was the coding sequence of proteins in the E3 region; in most cases, the mutations that were made to add the novel restriction sides did not result in a change in the coding sequence: however, when amino acid changes were made, they were conservative in nature.
It is thus important to point out a key advantage of such adenoviruses that have inserted in the E3 region a heterologous gene or genes, which is that such gene(s) will exhibit an expression pattern, both in terms of timing and degree of expression, similar to the endogenous adenoviral gene(s) that it replaces.

The adenoviral vectors of the instant invention can also incorporate a tissue specific promoter in a part of the E3 region that has been deleted that will drive the expression of another gene, preferably a negative selection gene. An example of a tissue specific promoter includes prostate specific antigen promoter. See, PCT/US95/14461. Examples of certain negative selection genes include cytosine deaminase, and thymidine kinase. Regarding cytosine deaminase, see, U. S. Patent Nos. 5, 358, 866, and 5,677,178.

For example, a HSV *tk* gene cassette may be operably linked immediately downstream of an E3 promoter. Frequently, it is desirable to delete a nonessential portion (i.e., for viral replication and packaging of the adenoviral genome to accommodate the negative selection cassette: thus a substantial portion of the E3 gene region may be deleted and replaced with a negative selection cassette such as an HSV *tk* gene operably linked to a tissue specific promoter (and enhancer) or other suitable promoter/enhancer. Alternatively, a negative selection gene may be operably linked to an adenovirus late region promoter to afford efficient expression of the negative selection gene product in cells expressing a replication phenotype characterized by transcription from late gene promoters.

Expression of the HSV *tk* gene in a cell is nor directly toxic to the cell, unless the cell is exposed to a negative selection agent such as gancyclovir or FIAU. Infected cells expressing a replication phenotype wherein a negative selection gene is substantially expressed may produce essentially no additional cytotoxicity until the negative selection agent (e.g., gancyclovir) is administered in an effective selective dosage, at which time the infected cells expressing the *tk* gene will be selectively ablated; thus negative selection can be used for enhanced cytopathic killing and/or to damp out further viral replication by killing cells exhibiting a replicative phenotype.

An example is an HSV *tk* gene cassette (Zjilstra et al. (1989) Nature 342:435: Mansour et al. (1988) Nature 336: 348; Johnson et al. (1989) Science 245: 1234. Adair et al. (198) Proc. Natl. Acad. Sci (U.S.A.) 86: 4574. Capecchi. M. (1989) Science 244:1288) operably linked to an appropriate promoter and/or enhancer with a polyadenylation site to form a *tk* expression cassette. The *tk* expression cassette (or other negative selection expression cassette) is inserted into the adenoviral genome, for example, as a replacement for a substantial deletion of the E3 region.

The adenoviral vectors of the instant invention that encode a desired protein can be used for transformation of a suitable mammalian host cell. Transformation can be by any known method for introducing polynucleotides into a host cell, including, for example packaging the polynucleotide in a virus and transducing a host cell with the virus or by transfection procedures known in the art, as exemplified by U.S. Patent Nos. 4,399,216, 4,912.040, 4,740.461. and 4,939,455. The transformation procedure used depends upon the host to be transformed. Methods for introduction of heterologous polynucleotides into mammalian cells are known in the art and include dextran-mediated transfection, calcium phosphate precipitation, polybrene mediated transfection electroporation, encapsulation of the polynucleotide(s) in liposomes and direct microinjection of the DNA into nuclei.

### Therapeutic Methods

Therapy of disease, preferably neoplastic disease, may be afforded by administering to a patient a composition comprising adenoviruses of the invention and further comprising a negative selection gene. Examples of the latter would include cytosine deaminase, and thymidine kinase.

Various human neoplasms may be treated with the invention adenoviral constructs, particularly in those instances where the E3 region of the virus encodes a protein useful for gene therapy of disease. An example would be a cytokine, preferrably an interleukin. For example but not by way of limitation, a human patient or nonhuman mammal having a bronchogenic carcinoma, nasopharyngeal carcinoma, laryngeal carcinoma, small cell and non-small cell lung carcinoma, lung adenocarcinoma, hepatocarcinoma, pancreatic carcinoma, bladder carcinoma, colon carcinoma, breast carcinoma, cervical carcinoma, ovarian carcinoma, or lymphocytic leukemias may be treated by administering an effective antineoplastic dosage of an appropriate adenovirus. Suspensions of infectious adenovirus particles may be applied to neoplastic tissue by various routes, including intravenous, intraperitoneal, intramuscular, subdermal, and topical. A adenovirus suspension containing about 10³ to 10¹² or more virion particles per ml may be inhaled as a mist (e.g., for pulmonary delivery to treat bronchogenic carcinoma, small-cell lung carcinoma, non-small cell lung carcinoma, lung adenocarcinoma, or laryngeal cancer) or swabbed directly on a tumor site for treating a tumor (e.g., bronchogenic carcinoma, nasopharyngeal carcinoma, laryngeal carcinoma, cervical carcinoma) or may be administered by infusion (e.g., into the peritoneal cavity for treating ovarian cancer, into the portal vein for treating hepatocarcinoma or liver metastases from other non-hepatic primary tumors) or other suitable route, including direct injection into a tumor mass (e.g., a breast tumor), enema (e.g., colon cancer), or catheter (e.g., bladder cancer).

The invention adenovirus mutants may be further evaluated by their capacity to reduce tumorigenesis or neoplastic cell burden in nu/nu mice harboring a transplant of neoplastic cells, as compared to untreated mice harboring an equivalent transplant of the neoplastic cells.

Adenoviral therapy using the instant invention E3 viruses may be combined with other antineoplastic protocols, such as conventional chemotherapy. Also, in the event that the instant E3 adenoviral vectors, or viruses elicit an immune response that dampens their effect in a host animal, they can be administered with an appropriate immunosuppressive drug.

### Propagation of Mutant Adenovirus

Adenoviral mutants of the invention typically are propagated as viral stocks in a cell line (e.g., the 293 cell line ATCC # CRL 1573, American Type Culture Collection, Rockville, MD; Graham et al. (1977) J. Gen. Virol. 36: 59, or A549 cells) which can provide certain desired viral functions, if needed, in trans to support replication and formation of infectious mutant virions.

### Formulations

Adenovirus E3 mutants may be formulated for therapeutic and diagnostic administration to a patient. For therapeutic or prophylactic uses, a sterile composition containing a pharmacologically effective dosage of one or more species of adenovirus mutant is administered to a human patient or veterinary non-human patient for treatment, for example, of a neoplastic condition. Generally, the composition will comprise about 10³ to 10¹⁵ or more adenovirus particles in an aqueous suspension. A pharmaceutically acceptable carrier or excipient is often employed in such sterile compositions. A variety of aqueous solutions can be used, e.g., water, buffered water, 0.4% saline, 0.3% glycine and the like. These solutions are sterile and generally, free of particulate matter other than the desired adenoviral virions. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. Excipients which enhance infection of cells by adenovirus may be included.

Adenoviruses of the invention, or the DNA contained therein, may be delivered to neoplastic cells by liposome or immunoliposome delivery; such delivery may be selectively targeted to neoplastic cells on the basis of a cell surface property present on the neoplastic cell population (e.g., the presence of a cell surface protein which binds an immunoglobulin in an immunoliposome). Typically, an aqueous suspension containing the virions are encapsulated in liposomes or immunoliposomes. For example, a suspension of adenovirus virions can be encapsulated in micelles to form immunoliposomes by conventional methods (U.S. Patent 5,043,164, U.S. Patent 4,957,735, U.S. Patent 4,925,661: Connor and Huang (1985) J. Cell Biol. 101: 582; Lasic DD (1992) Nature 355: 279: Novel Drug Delivery (eds. Prescott LF and Nimmo WS: Wiley. New York, 1989); Reddy et al. (1992) J. Immunol. 148: page 1585). Immunoliposomes comprising an antibody that binds specifically to a cancer cell antigen (e.g., CALLA, CEA) present on the cancer cells of the individual may be used to target virions, or virion DNA to those cells.

The compositions containing the present adenoviruses or cocktails thereof can be administered for prophylactic and/or therapeutic treatments of neoplastic disease. In therapeutic application, compositions are administered to a patient already affected by the particular neoplastic disease, in an amount sufficient to cure or at least partially arrest the condition and its complications. An amount adequate to accomplish this is defined as a "therapeutically effective dose" or "efficacious dose." Amounts effective for this use will depend upon the severity of the condition, the general state of the patient, and the route of administration.

In prophylactic applications, compositions containing the invention adenoviruses, or cocktails thereof, are administered to a patient not presently in a neoplastic disease state to enhance the patient's resistance to recurrence of a neoplasm or to prolong remission time. Such an amount is defined to be a "prophylactically effective dose." In this use, the precise amounts again depend upon the patient's state of health and general level of immunity.

Single or multiple administrations of the compositions can be carried out with dose levels and pattern being selected by the treating physician. In any event, the pharmaceutical formulations should provide a quantity of the antineoplastic adenoviruses of this invention sufficient to effectively treat the patient.

Antineoplastic adenoviral therapy of the present invention may be combined with other antineoplastic protocols, such as conventional chemotherapy.

### Uses of the Invention

It will be apparent, based on the discussion above, that the adenoviral vectors/viruses described herein have multiple uses including applications in gene therapy. For example, in one embodiment of the invention, a gene that encodes a medically useful protein may be cloned into the E3 region of the instant invention virions, and the virions used directly in gene therapy protocols to treat disease. In another embodiment of the invention, discussed above, such E3 mutant virions may also have deletions in the E1b region and have substituted therefore a gene with desirable properties. In either the E3 or E1B regions such genes might encode cytokines, including the interleukins, cell cycle regulatory proteins, including p16, or ras, or proteins that induce cellular suicide, or apoptosis, prodrug activators, including cytosine deaminase or thymidine kinase. Further, tumor necrosis factor alpha, interferon gamma, and mip-3 may be utilized.

The instant adenoviral vectors may also be used to express proteins that are useful immunogens, or as a vaccine, and to transform cells which do not ordinarily express a particular protein to thereafter express this protein. Cells expressing these molecules are useful as intermediates for making cell membrane preparations useful for binding assays, which are in turn useful for drug screening.

The Examples which follow are illustrative of specific embodiments of the invention, and various uses thereof. They are set forth for explanatory purposes only, and are not to be taken as limiting the invention.

### Example 1

### General Methods and Working Vectors

Methods for the construction and propagation of human adenovirus vectors are known in the art and will be understood to be applied in the Example presented below by the skilled practitioner of the art. Such would include the work of Hitt, M., et al Construction and propagation of human adenovirus vectors. In: Cell Biology: a Laboratory Handbook; J. Cells (Ed), Academic Press, N. Y. (1996); Graham, F. L. and Prevec, L. Adenovirus based expression vectors and recombinant vaccines. In: Vaccines: New Approaches to Immunological Problems. R. W. Ellis (ed) Butterworth. Pp. 363-390; and Graham, F. L. and Prevec, L. Manipulation of adenovirus vectors. In: Methods in Molecular Biology, Vol. 7: Gene Transfer and Expression Techniques. E. J. Murray and J. M. Walker (eds) Humana Press Inc., Clifton, N. J. pp 109-128, 1991. The materials and methods described in these articles were used below.

### Adenoviral Vectors:

Vectors based on pGEM (Promega Corp.) were modified and used to clone, subclone, and mutagenize the appropriate region E3 region of Ad5. This took advantage of existing restriction sites in the E3 region, and these are shown in Table 1.

**Table 1**

| Restriction sites present in Ad5 | |
|---|---|
| NdeI | 19549 and 31089 |
| SpeI | 27082 |
| EcoRI | 27295 and 30049 |
| SunI | 28390 |
| EcoRV | 27295 |
| KpnI | 28787 |
| MunI | 29355 |
| NotI | 29510 |
| XhoI | 29791 |
| HpaI | 30569 |

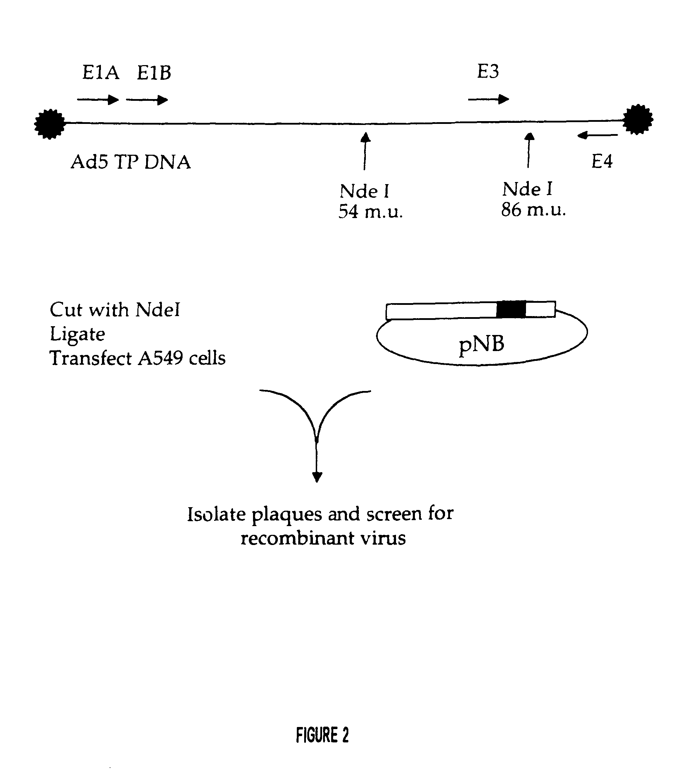

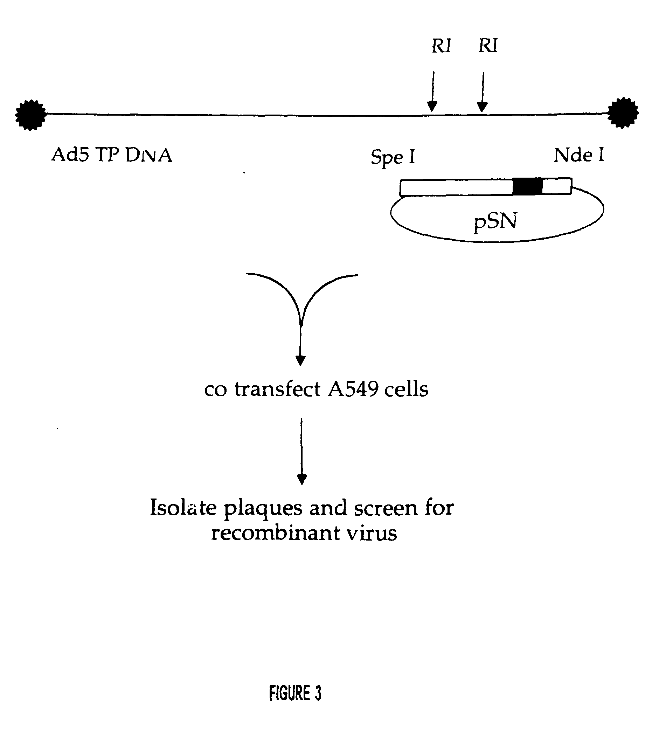

The vector designated pNB was made by inserting the NdeI fragment of Ad5, bases 19549 to 31089. into the NdeI site of pGEM5Zf+ (Promega Corp.). This was further subcloned by inserting the fragment from Ad5 SpeI (27082) to NdeI (31089) into the SpeI and NdeI sites in the multiple cloning site (MCS) in pGEM5Zf; this plasmid was designated pSN. The pNB vector was created for ease of virus construction; the Ad5 NdeI fragment can be isolated and ligated to NdeI-cut Ad5 TP-DNA. This can then be used to transfect cells as shown in figure 2. This construct is also amiable for virus construction using homologous recombination, since the area of overlap is extensive, as depicted in figure 3. The pSN plasmid was created because it contains the E3 region to be manipulated and because it is smaller than pNB and thus limits the variety of restriction sites.

In the case of pSN, the vector pGEM5 was further modified because it possesses 3 SspI sites (2199, 2384, 2408); SspI is one of the engineered sites in the E3 shuttle vector. By deleting the SspI sites in the vector, inserting genes into the E3 SspI site would be facilitated since it would not involve partial restriction digests. To delete the vector sites, the plasmid pGEM5 was cut with SspI and EcoRV (present in the MCS at base 51) and religated. Unfortunately, the SspI site at 2199 was unsatisfactorily deleted and found in the resultant altered vectors. So the resultant vector contains the deletion from SspI at 2384 to EcoRV at 51. The presence of this extra SspI site still requires partial restriction digestion when utilizing SspI in the E3 region, although the isolation of the correct fragment is simplified by the deletion of two of those sites. Note also that there is an SspI site in the E3 region at 30172; this resides in the same region that would be excised when the engineered SspI site is to be used. Thus, it does not affect the disgestion pattern, and it is of no consequence. This altered pGEM vector was used as the vector to insert the SpeI to NdeI region of Ad5, and for subsequent manipulations in the E3 regions and is indicated by pG.

### Example 2

### Construction of E3 Shuttle Vectors

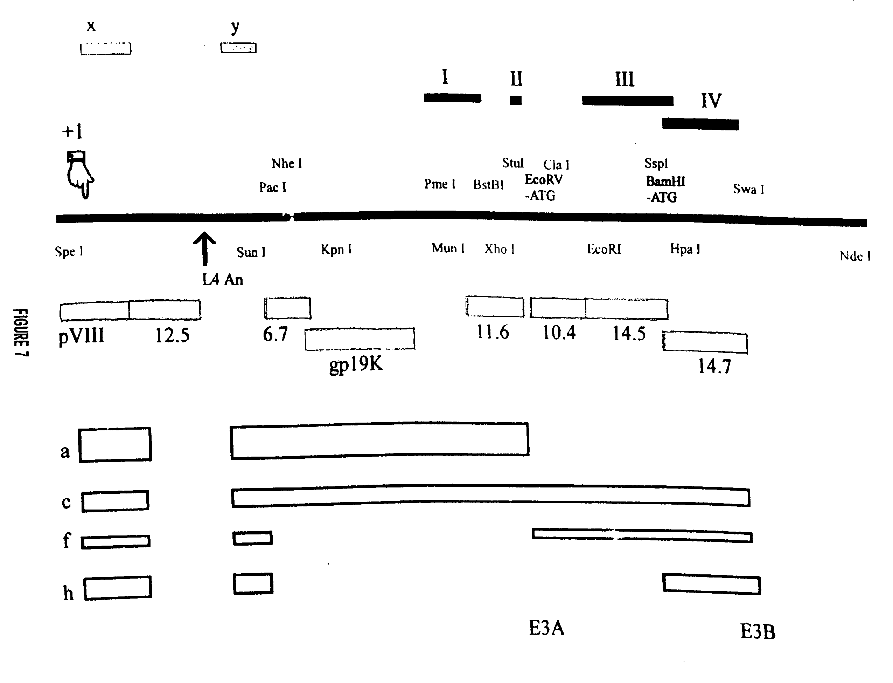

Using the above vectors, the following restrictions sites were engineered into the E3 region of adenovirus 5: PacI, ClaI, PmeI, SwaI, BamHI, BstBI, SspI, NheI, and StuI and EcoRV. Their relative positions in the E3 region are shown in figure 7. The restriction sites were carefully positioned so as to not knowingly disrupt critical splicing and polyadenylation signals (see, figure 1). Also considered was the coding sequence of proteins: in most cases, the coded amino acid was not changed, and when changes had to be made, they were conservative. After viral construction with these alterations, splicing and protein levels and functions were compared against the wild type Ad5 to confirm the functionality of the E3 genes in the shuttle vector.

Because of the position of the engineered sites, some mutations had to be performed sequentially. All of the oligonucleotide sequences used for mutagenesis and the exact location (position number in Ad5) are listed in the tables. All mutations were confirmed by restriction digests and all constructs were sequenced. Table 2 summarizes the restriction sites that were added to the E3 region of adenovirus 5.

**Table 2**

| Restriction sites added to the Ad5 E3. Numbers refer to the Ad5 genome. | |
|---|---|
| PacI | 28497 |
| NheI | 28532 |
| PmeI | 29310 |
| BstBI | 29484 |
| StuI | 29718 |
| EcoRV* | 29781 |
| ClaI | 29862 |
| SspI | 30377 |
| BamHI** | 30467 |
| SwaI | 30830 |

| | |
|---|---|
| * The start codon for 10.4K was altered with this mutation **The start codon for 14.7K was altered with this mutation | |

The sites PacI and ClaI were generated simultaneously by using mutant oligonucleotide PacC plus PacNC and ClaC plus ClaNC. respectively, using the Transformer Site-Directed Mutagenesis kit (Clonetech #K1600-1) as directed by the manufacturer. The sites PmeI and SwaI were constructed separately using PmeC plus PmeNC and SwaC plus SwaNC, respectively, using the QuickChange Sitc-Directed Mutagenesis kit (Stratagene #200518) exactly as described by the manufacturer. The Pmel site was cloned into the PacI/ClaI-containing plasmid by inserting the Pmel-containing KpnI-XhoI (natural Ad5 sites) fragment into this plasmid. To this construct, the SwaI site was inserted using the HpaI to NdeI fragment. This resulting construct was called pG-PPCS and used for the next round of mutagenesis.

### BamHI

All of the following mutations were created by PCR-based mutagenesis (See, Nucleic Acids Research 17:5404; 1989, and U.S. Patent Numbers 4,683.195 to Mullis et al. and 4.683.202 to Mullis) using Pfu polymerase, a high fidelity enzyme (Stratagene) and all fragments generated by PCR were subsequently sequenced and determined to be error-free. This procedure employed two sequential PCR reactions and then cutting the final product for insertion into the desired plasmid.

The BamHI site was generated with the oligonucleotides BamC and SwaNC in the first PCR and this product with PmeC in the second PCR, using the above-described plasmid pG-PPCS as the template. This fragment (the product of the second PCR) was digested with PmeI and SwaI to insert into pG-PPCS and this was termed pG-PPCS +B. It should be noted that this mutation also changes the 14.7K start codon to prevent premature initiation for any inserted gene at this site and that only two of the final four versions of the E3 shuttle vectors have the BamHI site.

### BstBI, NheI, and StuI

The BstBI site was created using BstBC and SwaNC in the first PCR and PmeC plus the first product in the second PCR. The template used was pG-PPCS. The second PCR product was digested with MunI and SwaI and inserted into pG-PPCS, resulting in pG-PPBCS. To make a version of this vector that contained the BamHI site, the fragment from ClaI to HpaI with this site was inserted to make pG-PPBCS+B.

The NheI site was made with NheC and PmeNC in the first PCR and SunC and the first product in the second PCR, using pG-PPBCS+/- B as the templates in separate reactions. This fragment was digested with PacI and PmeI and inserted into the two versions of the above described constructs, pG-PPBCS+/-B. This construct was called pG-PNPBCS+/-B. The StuI site was added by using StuC and SwaNC in the first reaction and PmeC and the first product in the second reaction, with pG-PPBCS+/-B as templates. The fragment was digested with MunI and SwaI and inserted into the two versions on the plasmid described in the previous paragraph (- or + BamHI) and these were called pG-PPBSCS or pG-PPBSCS+B, respectively. The new StuI and NheI sites were added together by digesting with the two plasmids with PacI and PmeI and inserting the fragment which contained the NheI site (from pG-PNPBCS) into the Stul-containing plasmid (pG-PPBSCS). This was done in plasmids which had or did not have the BamHI site and the resulting plasmids were called pG-PNPBSCS+B and pG-PNPBSCS. respectively.

### SspI and EcoRV

The last two mutations were made using both the above-described plasmids as templates, pG-PNPBSCS and pG-PNPBSCS+B. The SspI site was created with SspC and HpaNC primers in the first PCR and NheC and the product of the first PCR in the second PCR. This fragment was digested with XhoI and HpaI to insert into the parental plasmid, with or without the BamHI site. The EcoRV site was made using the EcoRVC and HpaNC primers in the first PCR and NheC and the first product in the second PCR. This fragment was cut with XhoI and HpaI to insert into the plasmids described in the above paragraph. The SspI and EcoRV sites were added together by cutting both BamHI-containing or BamHI-absent plasmids with PmeI and CIaI and inserting the EcoRV-containing fragment into the parental plasmids. Note the EcoRV site also changes the start codon for 10.4K to prevent premature initiation of genes inserted into the ClaI site at their 5' end. The ClaI site will be used, instead of the EcoRV site, for insertion of genes since the region with the EcoRV site is also involved in splicing. Deletion of this region might disrupt this event.

These final versions of the E3 shuttle vectors are depicted in Figs. 4 through 7. These are called pE3SV, pE3SV+V, pE3SV+B, and pE3SV+V+B, the differences being the presence or absence of the EcoRV and BamHI sites. These shuttle vectors are used for construction of all subsequent plasmids, be they insertion of foreign genes or deletion of the Ad5 E3 genes.

The oligonucleotides that were used to mutagnize the desired E3 region are shown in Table 3.

**Table 3:**

| Sequence of oligonucleotides used to mutagenize the E3 region of Adenovirus 5: | |
|---|---|
| SunC | CCTCTCCGAGCTCAGCTACTCCATCAG |
| PacC | GGAGGTGAGCTTAATTAACCCTTAGGG |
| PacNC | CCCTAAGGGTTAATTAAGCTCACCTCC |
| CD2-NdeC | GCTGCAAGTGCTGCACATGGGGCTGCATG |
| EcoRVC | GATTAAATGAGATATCATTCCTCGAG |
| HpaNC | GGCGGTGTCCGGTGGTATTACTGTCG |
| NheC | GGGTATTAGGCCAAAGGCGCAGCTAGCGTGGGG |
| StuC | CCCAAACAATGAAGGCCTCCATAGATTGG |
| SspC | CAGCTACTTTAATATTACAGGAGGAG |
| BstBC | GCGACCCACCCTTTCGAACAGAGATGACCAAC |
| BamC | GGAGACGACTGACACCCTGGATCCAGAAATGG |
| ClaC | CACATCGATGTAGACTGC |
| ClaNC | GCAGTCTACATCGATGTG |
| PmeC | TAGAATAGGGTTTAAACCCCCCGG |
| PmeNC | CCGGGGGGTTTAAACCCTATTCTA |
| SwaC | CTCAAAGATCTTATTCCATTTAAATAATAAA |
| SwaNC | WTATTATTTAAATGGAATAAGATCTTTGAG |
| CD-PacC | GTGAGCTTAATTAAGGCTAGCAATGTCGAATAACGC |
| CD-SwaNC | GTGAGCATTTAAATCAGTCGTTCAACGTTTGTAATC |

| | |
|---|---|
| All sequences written 5' to 3'. All changed bases are underlined. Inserted bases are in bold. | |

### Examples

### Construction of Virus and Controls

The final step was to insert these altered E3 regions into the pNB plasmids for construction of virus. To accomplish this, the plasmids pG-PPCS and pNB were cut with SpeI. The pNB has two SpeI sites: one in the Ad5 insert and one in the pGEM5 MCS. The fragment from pNB which contained a portion of the MCS and NdeI 19549 to SpeI 27082 was inserted into the SpeI-cut pG-PPCS plasmid. The orientation was confirmed to be correct, and the resulting plasmid termed pNB-PPCS. All of the final versions of the E3 shuttle vectors were cloned into this plasmid by inserting the PacI to SwaI region of the smaller plasmids into the larger pNB-PPCS. The resulting plasmids are designated pNB-E3SV, pNB-E3SV+V, pNB=E3SV+B. and pNB-E3SV+V+B. As shown in figure 2, these plasmids are then used to produce the corresponding adenoviruses termed E3SV. E3SV+V. E3SV+B. and E3SV+V+B.

It should be noted that there are three different sets of sites that can be used for inserting genes into the 6.7-gp 19K region. These are PacI and PmeI. SunI and MunI, and NheI and PmeI. The PacI site overlaps the y-leader an important sequence for translation of late gene products. Disruption of this sequence may abrogate its effect for certain applications. Therefore, another site. Nhel was inserted which does not overlap the y-leader. If no adverse affect is seen, then the SunI to MunI sites, naturally present in Add5. may be useful since it allows for a greater cloning capacity.

### Construction of Empty Controls

For controls, each of the E3 genes was deleted using the engineered sites. To do this the shuttle plasmids were cut with the following pairs of enzymes, filled in using T4 DNA polymerase, and religated: PacI and PmeI. SunI and MunI. Nhel and PmeI. BstBI and StuI (all in pG-E3SV). ClaI and SwaI (in pG-E3SV÷V), ClaI and SspI (in pG-E3SV+V), and BamHI and SwaI (in pG-E3SV+B). These were all built into the pNB vector for virus construction.

### Examples 4

### Construction of CD Plasmids

To test the shuttle vector system for its therapeutic use, the E. coli gene cytosine deaminase (CD) was used because of its prodrug capabilities. CD was obtained from ATCC (#40999. plasmid pCD2) and the CD gene was amplified from this plasmid as follows. It should also be noted that the CD gene contains an Ndel restriction site; and because we intended to use this particular enzyme to cut NdeI sites (the Ad5 NdeI sites 19549 and 31089) that would be present in the final plasmids, it was necessary to remove the NdeI site in the CD gene by PCR mutagenesis. This technique is the same one that was used to engineer in the new restriction sites into the E3 region, using the high-fidelity Pfu polymerase. Tables 3 and 4 shows oligonucleotides used to amplify the CD gene.

Briefly, a conservative mutation was made in the NdeI site, changing the base T to a C. The primers for the first PCR reaction were CD-NdeC and SwaCDNC. The plasmid pCD2 was used as a template. This product, along with the same template and primer CD-PacC, were used for the second PCR reaction. This accomplished two goals: it altered the Ndel site and added restriction sites PacI and SwaI to the 5 and 3' ends, respectively. This final PCR product was cut with PacI and SwaI: the shuttle vector, E3SV was also digested with PacI and SwaI. The fragments were gel purified using the Qiagen gel extraction kit and then ligated together using NEB T4 DNA Lipase. The E.coli strain XL- was transformed with the ligation mix, plated on ampicillin-containing plates for selection, and colonies were picked and cultured. The DNA was isolated and then screened by restriction digest to check for correct insertion and deletion. The clones which appeared correct were then sequenced through the entire CD gene and surrounding vector to verity that no unwanted mutations had taken place. This correct and verified clone was called pG-CDPacSwa and used in subsequent PCR amplifications where the CD gene was amplified for insertion into other regions. It should be noted that the CD gene contains a bacterial start codon, GTG. In all 5 primers, the start codon was included and changed to the eukariotic codon, ATG.

The other CD-containing vectors were created by designing the appropriate primers which possess the desired restriction site at the 5' or 3' end of the gene; the 5' primer always containing the ATG start codon. The CD gene was inserted into E3 regions using the following restrictions sites: BstBI to StuI, NheI to MunI, NheI to Pmel. PacI to PmeI, SunI to MunI, ClaI to SwaI, and BamHI to SwaI. The plasmids were named: pG-CDBstStu, pG-CDNheMun, pG-CDNhePme, pG-CDPacPme, pG-CDSunMun, pG-CDClaSwa, pG-CDBamSwa, respectively. All primers used are listed in Table 3 and the template was always the confirmed plasmid pG-CDPacSwa. The CD gene in the ClaI to SwaI region was inserted into the E3SV+V plasmid; the CD gene in the BamHI to SwaI region was inserted in the E3SV+B plasmid. All insertions were sequenced completely to ensure that no unwanted mutations had taken place and that the CD gene was inserted correctly.

To allow for virus construction, the constructs pG-CDClaSwa, pG-CDPacSwa, and pG-CDBamSwa were digested with PacI and SwaI and the CD gene-containing fragment was gel-purified. At the same time, the vector pNB-E3SV was also cut with PacI and SwaI and gel-purified. They were ligated, bacteria was transformed, and colonies were screened and selected for future virus construction.

Several points are predicted from the viruses that result from the above E3 insertions. First, the construct which remove portions of the y-leader, as in pG-CDSunMun, may cause an adverse effect on the course of the infection, as discussed above. This may also be true of genes inserted into the PacI site, although less of the y-leader is deleted. Another prediction is that inserts into the 11.6K region, as in pG-CDBstStu, may result in a greatly attenuated infection. As has been published, deletion of the 11.6K protein (ADP or adenovirus Death Protein) does not allow the infected cells to lyse at the proper time, compared to wild type infection. In this case the cell continues to metabolize, the virus production per cell is higher, and the cell becomes basically a factory for the foreign gene. Also, since ADP is synthesized in large quantities using the major late promoter during the late phase of infection, the foreign gene inserted into the region is expected to have the same expression characteristics. The results obtained with these viruses will be discussed below.

### Example 5

### Construction of TNF Plasmids

The plasmid containing the murine tumor necrosis factor (mTNF) gene was obtained from ATCC (#63169). This sequence contains the entire mTNF gene including the coding region for the prosequence. The mTNF gene was amplified from this plasmid by PCR and gel purified. The vector pGE3SV was cut with BstBI and StuI and gel-purified. At the same time, another vector, pG-E3SV+V was cut with ClaI and SwaI and gel purified. The purified PCR product was inserted into each of these vectors, allowed because of compatible ends. These constructs were called pG-mTNFBstStu and pG-mTNFClaSwa, respectively.

Using the ATCC plasmid as a template once again, mTNF gene was amplified by PCR. The plasmid pG-E3SV+B and the PCR product were cut with BamHI and SwaI, gel purified, and ligated together. This construct was called pG-mTNFBamSwa. All constructs were sequenced extensively to check for unwanted mutations.

### Example 6

### Construction of CD and TNF Viruses

To build the above constructs into the Ad5 genome, BstLink TP-DNA was used since it offers advantages described previously. The plasmid construction for this is described in Example 8. The construction of the virus was prepared just as described for these recombinant viruses, using Ad5 wild type TP-DNA. Note that all transfections were performed on 6 cm dishes and in duplicate; the quantities described here are per each 6 cm dish.

Methods: the viruses E3-CD-PacPme (Onyx 301), E3-CD-NhePmc (Onyx 302), E3-CDSunMun (Onyx 303), E3-CD-NheMun (Onyx 304), E3-CD-BstStu (Onyx 305), and E3-mTNF-BstStu (Onyx 320) were made as follows: first, 0.5 micrograms of BstLink TP-DNA and ten micrograms of plasmid were cut with EcoRI (20 units) at 37 degrees for 5 hours. (An overabundance of plasmid DNA was used to allow approximately five micrograms of the actual insert DNA per transfection) At this point, the TP-DNA was left to digest at room temperature overnight while the cut plasmids were run on a 1% agarose gel overnight. The inserts were gel-purified using the Qiagen gel extraction kit. Ligation reactions consisted of the cut TP-DNA and the purified fragments with 10 units of high concentration T4 DNA Ligase (Boehringer Mannheim) overnight at 16 degrees. This reaction mixture was used directly for transfection.

For construction of viruses CDPacSwa, mTNFClaSwa, CDBamSwa, mTNFBamSwa, because these mutations in the E3 region lie outside of the EcoRI restrictions sites, a different method was used; homologous recombination. The quantities of DNA and TP-DNA are the same as above. The TP-DNA BstLink was cut with BstBI for the transfection. The CD-containing plasmids were cut with SpeI and Ndel and the mTNF-containing plasmids were cut with PacI and NdeI. The fragments were gel-purified and eluted in water. For the transfections, the cut TP-DNA and the isolated fragments were used without any further manipulations.

Transfection Procedures: For transfections, A549 cells were plated onto 6 cm dishes the preceding day so that they would be approximately 70 to 80% confluent the day of the transfection. To transfect these cells, 2 solutions were made then subsequently mixed. Solution A contained the ligation mixture and 300 microliters of OptiMEM (Life Technologies) per 6 cm dish. Solution B contained 300 microliters of OptiMEM and 13 microliters ofLipofectamine (Life Technologies). These two solutions were added together, mixed gently, and allowed to incubate at room temperature for 30 to 45 minutes. Near the end of this incubation time, the cells were washed with warm OptiMEM and 2.4 milliliters of OptiMEM was added to each of the mixtures. This final 3 ml mix was then added directly to the washed cell monolayer and incubated at 37 degrees for 5 hours. Then 3 ml of DME containing 20% FBS was added to each dish without removing the transfection mix, bringing the final serum concentration to 10%. This was allowed to incubate at 37 degrees overnight. The cells were overlaid with 8 ml of DME/ 2% FBS/1.0% agar noble (Difco). Five days after this overlay, another overlay (5 ml) was added which also contained 0.3 % neutral red (Life technologies) to help visualize the plaques.

Propagation and confirmation of virus mutants: as plaques appeared (10 to 20 days after transfection), they were isolated as agar plugs using a sterile Pasteur pipette. To propagate the virus present in the agar plugs, 3.5 cm plates were seeded with A549 cells in DME/10% FBS on the previous day. The day of the infection, the medium was changed to DME/2%FBS and the isolated plaques were added to the cells. The infections were checked daily for CPE (cytopathic effect, where the cells become rounded up and detach from the plate as a result of the virus infection), which usually occurred 3 to 5 days after infection. The entire medium and cells were collected and frozen at -20 degrees. To check for virus mutation, 200 microliters of the cell and medium mix was used to isolate the viral DNA (along with cellular DNA) using the Qiagen Blood kit. This purified DNA was checked by PCR using primers which corresponded to the CD gene itself or the flanking E3 region. Once the PCR of the recombinant virus DNA was shown to produce a correct size fragment, further characterization included cutting the PCR fragments with restriction enzymes for patterns unique to CD or mTNF and also by sequencing the PCR product. Also to confirm correct virus was obtained, Hirt analysis was performed.

The correct viruses were expanded by infecting a T150 of A549 cells with 500 microliters of the CPE obtained from the 3.5 cm dish. This was allowed to proceed to full CPE (when over 75% of the cells are no longer attached to the flask surface), which occurred in approximately 3 days. Then 7.5 ml of this cell and medium mixture was used to infect a 3-liter spinner of KB cells and was CeCl-banded. Plaque assays were performed to determine the infectious particles per unit volume.

The viruses were named at this point in such a way to make it obvious to tell what insert has been added and where the insert is placed. Numbers for easy of reference was also assigned to each virus and appears in parenthesis. Their names are E3-CD-PacPme (Onyx 301), E3-CDNhePme (Onyx 302), E3-CD-SunMun (Onyx 303), E3-CD-NheMun (Onyx 304), E3-CDBstStu (Onyx 305), and E3-mTNF-BstStu (Onyx 320).

CD assay: To assay for cytosine deaminase (CD) activity, the reaction was performed similar to that as described in Rogulski et al 1997. Briefly, A549 cells were seeded into 10 cm plates so that they were about 70 to 80% confluent on the day of infection (about 2 to 4 million cells per plate). The cells were infected at an MOI (multiplicity of infection) of 10 pfu (plaque forming units) per cell for each of the E3-CD viruses. Ad5 and mock infected were included as controls. For the infection, the proper volume of virus was suspended in 2 ml of DME per 10 cm plate and then added to the cell monolayer. After one hour, 8 ml of DME/2% FBS medium was added to each plate. At various times post-infection (4, 8, 12, 24, 36, 48, 60, 72, 84, 96, 120 hours), the cells were rinsed, 1ml of cold PBS was added, cells were scraped (using disposable cell scrapers) and pelleted into 1.5 ml eppendorf tubes. All PBS was removed and the cell pellets were flash-frozen in dry ice/ethanol and stored at -80 degrees. 200 microliters of assay buffer (100 mM Tris/HCl (pH 8.0) 1 mM EDTA, 1mM B-mercaptoethanol) was added to each pellet and the cells were lysed by 4 freeze/thaw cycles. The lysates were cleared by centrifugation at full speed for 5 minutes at 4 degrees. The quantity of protein was determined by a Bradford assay using Bio-rad reagents. For the enzyme assay, either 5 micrograms or 0.6 micrograms of protein from each sample were used, along with 2.5 mM [2-14C]-cytosine (1 microCurie; 5 microliters; Moravek Biochemicals, #MC131) and assay buffer to bring the reaction volume to 10 microliters. The reaction was allowed to proceed for one hour at 37 degrees C. To quench the reaction, 10 microliters of cold cytosine/uracil (0.4 mg/ml each) was added. Ten microliters from each sample was spotted onto a thin layer chromatagraphy plate (Baker #7009-04) and then placed in an equilibrated tank with 1-butanol-water (86%/14%). After allowing the solvent front to approach the top of the plate (about 2 hours), the plate was allow to dry and then exposed to film. This autoradiogram was then scanned for the figures. Murine TNF alpha assay: The A549 cells were plated onto 6 cm plates so that they would be approximately 80% confluent for the infection. The infection was performed as described above at an M.O.I. or 10. At various time points the medium was removed and replace with 3 ml of fresh DME/2% FBS. This was incubated at 37 degrees for one hour. After that interval, a one ml aliquot of the medium was removed and stored frozen at -80 degrees until all samples were collected. The medium was replaced on each plate so the final volume was 4 ml until the next time point. The mTNF that was secreted into the medium was assayed by an ELISA assay (Biosource #KMC3012). Each sample was determined in duplicate and each time point was collected from 2 different plates of infected cells. Western Blot analysis: For western blot analysis, A549 cells on a 6 cm plate were infected at an M.O.I. of 10. At various times postinfection, the cells were scraped and collected as described above. The cells pellet was stored at -80 degrees. Three hundred microliters of lysis buffer was added to each sample, freeze/thaw 3 times, and passed through a 22-gauge needle. A Bradford assay was performed to determine quantity of protein. Ten micrograms of total protein was loaded onto a 14% SDS gel and electrophoresed. The proteins were transferred to PVDF transfer membrane blocked with 3% dry milk in PBS, and blotted with the appropriate antibody. The antibodies for the E3 proteins and for pVIII (a protein made during the late times of infection) were polyclonal rabbit antibodies. These were used at 1:400. The antibody for murine TNF was obtained from R and D systems and was used at 0.1 micrograms per ml. The appropriate secondary antibodies were used and then visualized using the ECL system (Amersham).

In addition to western analysis of cell lysates, the medium that was collected at hourly intervals was analyzed by western blots using the same anti-mTNF antibody using 25 microliters of medium loaded per lane.

**Table 4: Oligonucleotides used to amplify the CD gene.**

| | |
|---|---|
| PacCD | GTGAGCTTAATTAAGGCTAGCAATGTCGAATAACGC |
| PmeCD | GTGAGCGTTTAAACAGTCGTTCAACGTTTGTAATCG |
| NheCD | GGCCGCTAGCGGCTAACAATGTCGAATAACGC |
| SunCD | GTGAGCCGTACGAGGCTAGCAATGTCGAATAACGC |
| MunCD | GTGAGCCAATTGCAGTCGTTCAACGTTTGTAATCG |
| BstBICD | GCGCTTCGAAGTGGAGGCTAACAATGTCGAATA |
| StuICD | GGCCAGGCCTCTAAGCTCGCTGTAACCCAGTCG |

All sequences are written 5' to 3'. All changed bases are underlined. Inserted bases are in bold.

### Example 7

### Viral Expression of CD or mTNF

CD-containing viruses:_E3-CD-PacPme (Onyx 301), E3-CD-NhePme (Onyx 302), E3-CDSunMun (Onyx 303), E3-CD-NheMun (Onyx 304), also referred to as 301, 302, 303, and 304, respectively. The cell line A549 was infected with each of the viruses 301, 302, 303, and 304 at a M.O.I.(multiplicity of infection) often. At the designated times post infection (p.i.), samples were harvested as described in methods section for assay of CD activity. Also at each time point, a picture was taken of the cells to show phenotypic differences.

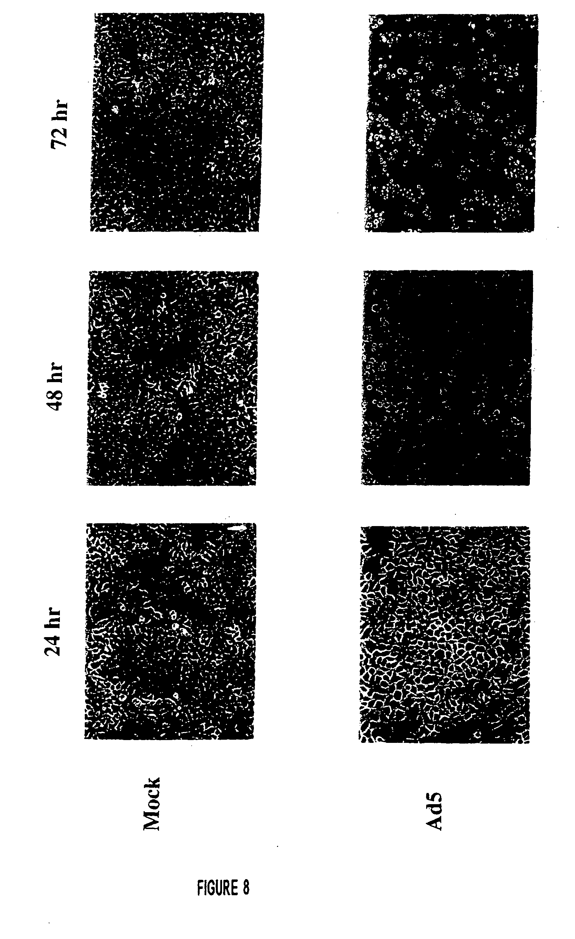

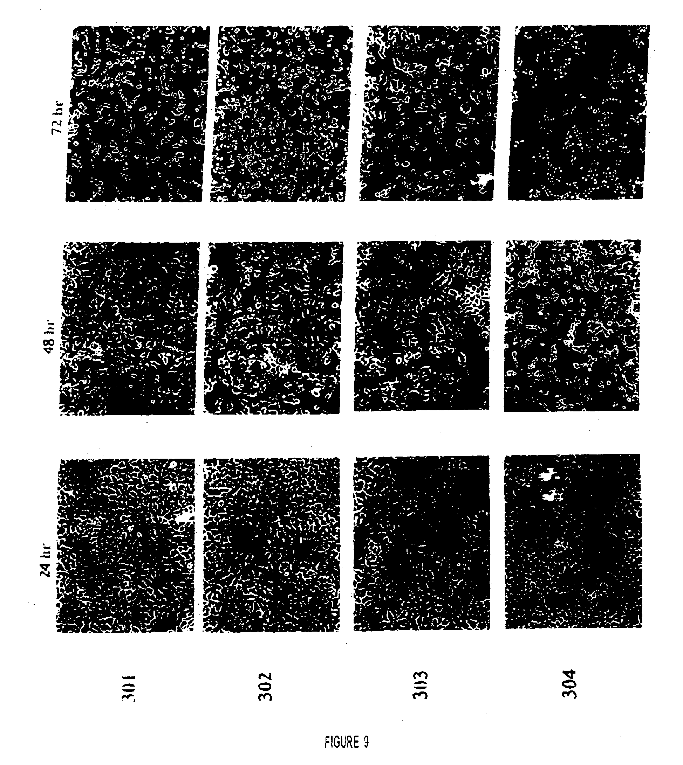

Figure 8 shows control mock infected and Ad5-infected cells, and figure 9 shows cells infected with viruses with CD inserted into the gp19K region, that is, viruses 301, 302, 303, and 304. The wild type infection procedes normally and shows almost total CPE by 48 hr postinfection. The 304 virus shows near wild type levels of CPE at 48 hr p.i., while 303 shows a slightly attenuated infection. The other two viruses, 301 and 302, show an intermediate phenotype. Interestingly, 303 is the CD substitution using the SunI site and as predicted, the infection is slower than wild type likely due to the deletion of part of the y-leader, abrogating it's addition to late messages and probably lowering their efficiency of translation. The viruses 301 and 302 lag behind Ad5 only slightly since most, in the case of 301, or all, in the case of 302, of the y-leader is intact.

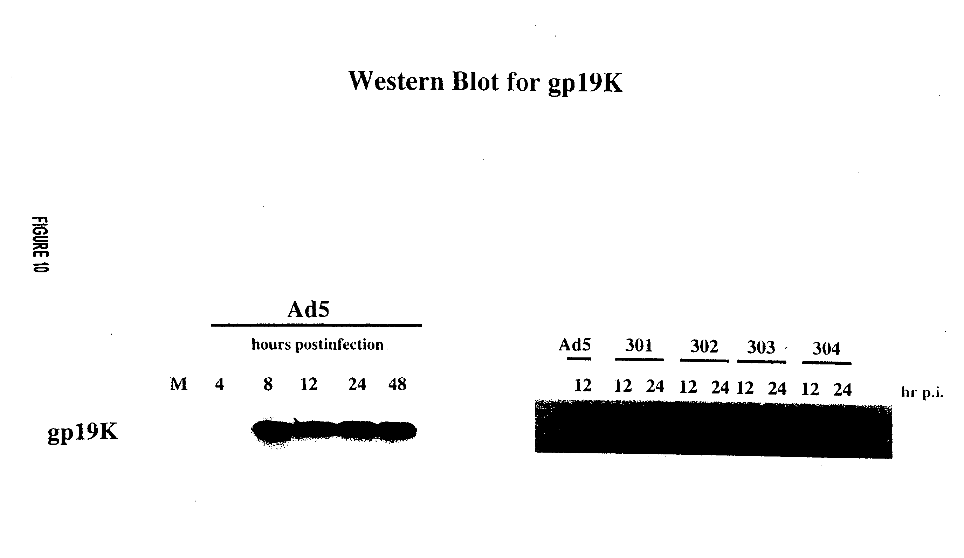

Experiments show that the time of expression of heterologous genes inserted in the E3 viral constructs of the instant invention is similar to the endogenous viral genes that they replace. The viruses 301, 302, 303, and 304 are substitutions ofgpl9K. As shown in fig 10, gp19K synthesis begins between 4 and 8 hours postinfection, using Western blot analysis. This is similar to published values. It is apparent at 48 hr p.i. by Western blot. Also shown in fig 10 is that the CD viruses do not make gp19K, as predicted since the gene is deleted.

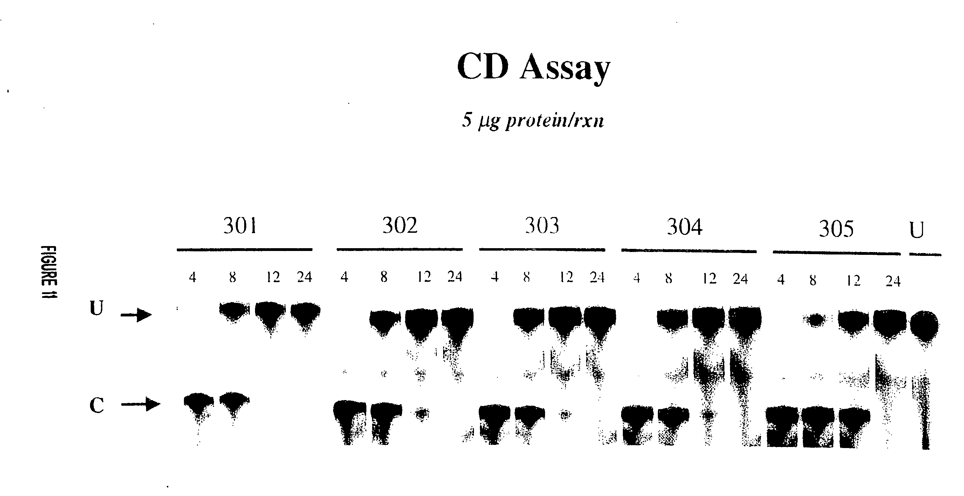

To see if the timing of the expression of CD synthesis is similar to gp19K synthesis, the CD protein was analyzed by a functional assay. The CD assay was set up as described in the previous section. Although the protein is extremely stable, the assay is not as sensitive as a Western blot. For that reason, two different assays were used to look at CD activity using two different amounts of protein in each reaction. To check when CD is first expressed, 5 micrograms of total protein was used per reaction. The results are shown in fig 11. As with gp19K, the CD activity is seen as early as 8 hr p.i. in viruses 301 through 304. This validates that endogenous expression time is similar to the inserted CD gene.

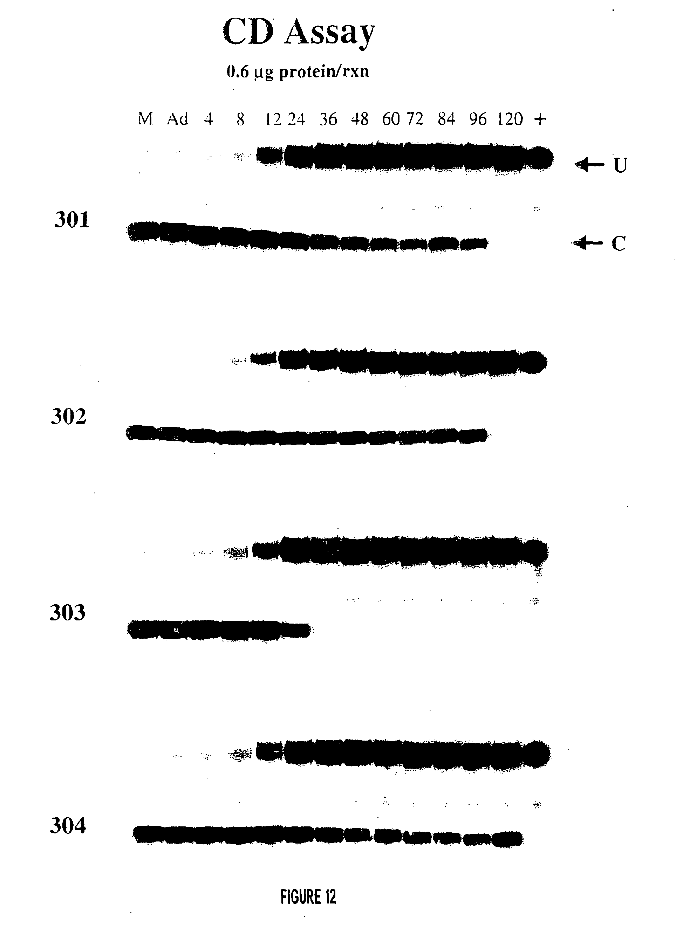

To get an idea of the amount of CD protein being synthesized from each position, 0.6 micrograms were used for each reaction and the results are shown in fig 12. A smaller amount of protein was used in order to see incomplete conversion of the substrate to compare between the viruses. Fig 12 shows that 301, 302, and 304 synthesize similar amounts of CD. On the other hand, 303 shows total conversion of substrate at about 24 hours, indicating that there is proportionally more CD present. Therefore we conclude that this virus probably makes more CD than the others.

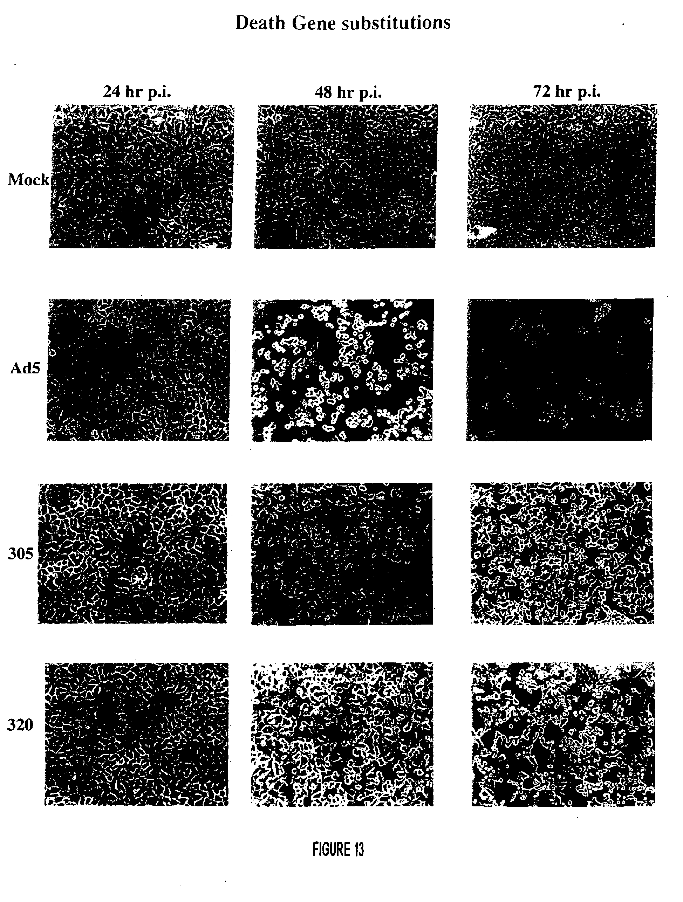

ADP substitutions: CD and mTNF: The E3-Adenovirus Death Protein (ADP) gene was replaced with either CD or mTNF, as described above. It is known that ADP deleted viruses do not lyse infected cells at the expected time compared to wild type. Thus, the gene is thought to be important in virus release. As with the ADP deletion viruses, the invention viruses which replace other genes in this region show a similar phenotype. This is shown in fig 13 and fig. 14. At 72 hr p.i. when the wild type infection shows total CPE, virus 305 (CD insertion) and virus 320 (mTNF insertion) exhibit significant CPE. The infection doesn't reach total CPE until 96 hr p.i. (fig 14). If the medium is changed every 24 hrs, the cells remain attached and exhibit an almost normal phenotype even at 120 hr p.i.. It is not until after 164 hr p.i. (7 days) that the cells appear to show classic CPE and come off the dish. This is a key observation and will be useful in a therapeutic sense since even if infected cells do not lyse immediately, they will nevertheless continue to express the heterologous gene of interest.

ADP is a late protein synthesized from the major late promoter and expressed at high levels during the late phase, that is, after DNA replication. To determine if inserted foreign genes show similar kinetics, a CD assay was performed on 305, and analysis for mTNF was performed on 320. As shown in fig 11, 305 does not show substantial CD activity until 12 hr p.i., after the time when the virus has entered into the late phase. Also contrasted in fig 11 is the comparison with CD in the gp 19K region, an early region. Clearly there is a difference in the timing that the protein is synthesized.

To compare amount of CD activity, a CD assay was performed using 0.6 micrograms of total protein. Upon visual inspection and comparison to fig 12 (experiments were done at the same time), it appears that 305 does not synthesize quite as much as 301-304. But it should be noted that the 305-infected cells have an attenuated course of infection.

As a check for late stage of infection, western blot analysis was performed on cell lysates and blotted with an antibody to a late structural protein, pVIII and for ADP (11.6K). Both 11.6K and pVIII expression is seen in Ad5 and 320 infections at 24 hr p.i., indicating that the virus has entered the late phase between 12 and 24. Also observed is that 305 does not make ADP, as expected since it does not contain the gene.

CD inserted in place of ADP exhibits a similar expression time as ADP. A similar observation was made with Onyx 320, which has mTNF inserted in place of ADP. Briefly, cells were infected and lysates made at the different times p.i. Western blot analysis was performed on these lysates, using both mock-infected and Ad5-infected cells as controls. It was shown that intracellular mTNF expression is not seen until after 24 hours p.i. This is consistent with our previous findings that foreign genes which replace endogenous adenoviral genes exhibit similar expression patterns.

### Example 8

### Construction of BstLink Virus/TP-DNA

Insertion of a gene of choice is an extensive process since it involves cloning into the smaller plasmids first and then adding this into the larger, pNB-based vectors. This is almost always due to extensive number of restriction endonuclease sites present in the larger pNB plasmid relative to the smaller pSN-based plasmids. The instant virus makes cloning easier because it avoids isolation of partial-cut fragments, which are often hard to separate from unwanted fragments, and working with smaller plasmids usually results in a higher DNA yield. However, their use for cotransfections for virus construction is difficult because it allows a limited amount of overlapping sequences necessary for homologous recombination For example, there is only a 240 base pair overlap at the 5' end when cutting TP-DNA with EcoRI, the standard method. Thus, to increase the region of overlap, a virus called BstLink was created as follows. The empty control known as pG-Bst→Stu was used because of the selection advantage it offers. This deletes the 11.6K death gene which results in much smaller plaques. Therefore when selecting recombinant viral plaques that should contain the 11.6K gene (or another death gene), the phenotypic difference between wild type (small plaques) and the recombinant (larger plaques) will make selection of recombinants clearer. This plasmid was digested with MunI, filled in with T4 DNA polymerase, and a BstBI linker was added by ligation. This restriction site was chosen because it is not present anywhere else in the Ad5 genome. The working objective is to build this construct (an E3 region with no 116.K and containing the additional BstBI site) into a virus (any background), prepare TP-DNA from the virus, and then use this for virus construction. This is done by cutting the TP-DNA with BstBI and cotransfecting with the E3 plasmid with the desired alterations. This increases the homology at the 5'end to 2273 base pairs. Time will be conserved as plasmids don't need to be further constructed from the small plasmids to the large plasmids. Also recombinant viruses is easier to select base on phenotypic differences. The plaques still have to screened for the mutations but the proportion of correct virus clones is predicted to be higher.

The invention now being fully described, it will be apparent to one of ordinary skill in the art that many changes and modifications can be made therero within the scope of the appended claims.

## Claims

1. A recombinant adenoviral vector comprising restriction sites in the E3 region of said vector selected from the group consisting of Pac1, Clal, Pme1, Swa1, BamH1, BstB1, Ssp1, Nhe1, Stu1 and EcoRV,
wherein said restriction sites have relative positions as shown in Figures 4 to 7;
wherein restriction sites are introduced by way of mutation preferably without changing the coding sequence, but where amino acid changes are made they are conservative in nature; and
wherein restriction sites are positioned so as not to disrupt critical splicing and polyadenylation signals;
wherein restriction sites facilitate partial or total deletion of an adenoviral gene or genes contained in said E3 region, and substitution of said partial or total deletion of said adenoviral gene or genes with a heterologous gene,
wherein said heterologous gene exhibits an expression pattern, both in terms of timing and degree of expression, similar to the endogenous adenoviral gene it replaces;
wherein said vector includes restriction sites in the early region genes of the E3 region that encode the 6.7k and gp19k proteins and/or
wherein said vector includes restriction sites in the early region genes of the E3 region that encode the 10.4k, 14.5k and the 14.7k proteins.

2. A recombinant adenoviral vector according to claim 1 further comprising a deletion in an E1A or E1B region of said adenoviral vector, and wherein said E1B region encodes a 55k protein.

3. A recombinant adenoviral vector according to any one of the preceding claims comprising a heterologous gene substituted for said partial or total deletion of said adenoviral gene or genes of the E3 region, wherein said heterologous gene exhibits an expression pattern, both in terms of timing and degree of expression, similar to the endogenous adenoviral gene it replaces.

4. A recombinant adenoviral vector according to claim 2 or claim 3 wherein said E1B or said E1A region deletions are substituted with a gene that encodes a heterologous protein.

5. A recombinant adenoviral vector according to any one of the preceding claims wherein said heterologous protein is selected from the group consisting of tumour necrosis factor alpha, interferon gamma, an interleukin, a cell suicide protein and mip-3.

6. A recombinant adenoviral vector according to any one of claim 1 to 4 wherein the heterologous protein is a negative selection gene.

7. A recombinant adenoviral vector according to claim 6 wherein the negative selection gene is selected from the group consisting of cytosine deaminase and thymidine kinase.

8. Use of a recombinant adenoviral vector according to any one of claims 1 to 7 in the preparation of a medicament for treating cancer in a mammal.

9. Use according to claim 8 wherein said medicament is for administration in association with a chemotherapeutic or immunosuppressive.

10. A method of producing a recombinant adenoviral vector for use in expressing a heterologous gene, said method comprising engineering a restriction site into the E3 region of said vector selected from the group consisting of Pac1, Cla1, Pme1, Swa1, BamH1, BstB1, Ssp1, Nhe1, Stu1 and EcoRV;
wherein said restriction sites have relative positions as shown in Figures 4 to 7;
which restriction site enables the partial or total deletion of an adenoviral gene or genes contained in said E3 region, and substitution of said partial or total deletion of said adenoviral gene or genes with a heterologous gene; said restriction site being positioned such that said heterologous gene exhibits an expression pattern, both in terms of timing and degree of expression, similar to the endogenous adenoviral gene it replaces without disrupting critical splicing and polyadenylation signals;
wherein any amino acid changes are conservative in nature;
wherein said restriction site is engineered into the early region genes of the E3 region that encode the 6.7k and gp19k proteins; and/or
wherein said restriction site is engineered into the early region genes of the E3 region that encode the 10.4k, 14.5k and the 14.7k proteins.

11. A method according to claim 10 further comprising the step of introducing a deletion in a E1A or E1b region of said adenoviral vector, and wherein said E1b region encodes a 55k protein.

12. A method according to claim 10 or claim 11 further comprising the step of substituting a heterologous gene for said partial or total deletion of said adenoviral gene or genes, wherein said heterologous gene exhibits an expression pattern, both in terms of timing and degree of expression, similar to the endogenous adenoviral gene it replaces.

13. A method according to claim 11 further comprising substituting said E1b or said E1A region deletions with a gene that encodes a heterologous protein.

14. A method according to any one of claims 10 to 13 wherein said heterologous protein is selected from the group consisting of tumour necrosis factor alpha, interferon gamma, an interleukin, a cell suicide protein and mip-3.

15. A method according to any one of claim 10 to 13 wherein the heterologous protein is a negative selection gene.

16. A method according to claim 15 wherein the negative selection gene is selected from the group consisting of cytosine deaminase and thymidine kinase.

## Patentansprüche

1. Rekombinanter Adenovirusvektor, umfassend Restriktionsstellen in der E3-Region des Vektors, die aus der aus Pac1, Ca1, Pme1, Swa1, BamH1, BstB1, Ssp1, Nhe1, Stu1 und EcoRV bestehenden Gruppe ausgewählt sind,
worin die Restriktionsstellen an relativen Positionen liegen, wie sie in den Fig. 4 bis 7 gezeigt werden;
worin Restriktionsstellen mittels Mutation, vorzugsweise ohne Veränderung der kodierenden Sequenz, eingeführt sind, wobei aber, wenn Aminosäureänderungen erfolgt sind, diese konservativer Natur sind; und
worin Restriktionsstellen so positioniert sind, dass sie entscheidende Spleiß- und Polyadenylierungssignale nicht unterbrechen;
worin Restriktionsstellen die partielle oder vollständige Deletion eines oder mehrerer in der E3-Region enthaltener Adenovirusgene und Substitution der partiellen oder vollständigen Deletion des einen oder der mehreren Adenovirusgene durch ein heterologes Gen erleichtern,
worin das heterologe Gen ein Expressionsmuster aufweist, das sowohl den Zeitablauf als auch das Ausmaß der Expression betreffend ähnlich ist wie jenes des endogenen Adenovirusgens, das es ersetzt;
worin der Vektor Restriktionsstellen in den Genen der frühen Regionen der E3-Region umfasst, die für das 6,7k- und das gp19k-Protein kodieren, und/oder
worin der Vektor Restriktionsstellen in den Genen der frühen Regionen der E3-Region umfasst, die für das 10,4k-, das 14,5k- und die 14,7k-Protein kodieren.

2. Rekombinanter Adenovirusvektor nach Anspruch 1, der weiters eine Deletion in einer E1A- oder E1B-Region des Adenovirusvektors umfasst, worin die E1 B-Region für ein 55k-Protein kodiert.

3. Rekombinanter Adenovirusvektor nach einem der vorangegangenen Ansprüche, umfassend ein heterologes Gen, das anstelle der partiellen oder vollständigen Deletion des einen oder der mehreren Adenovirusgene der E3-Region substituiert ist, worin das heterologe Gen ein Expressionsmuster aufweist, das sowohl den Zeitablauf als auch das Ausmaß der Expression betreffend ähnlich ist wie jenes der endogenen Adenovirusgens, das es ersetzt.

4. Rekombinanter Adenovirusvektor nach Anspruch 2 oder Anspruch 3, worin die Deletionen der E1B- oder der E1A-Region durch ein Gen substituiert sind, das für ein heterologes Protein kodiert.

5. Rekombinanter Adenovirusvektor nach einem der vorangegangenen Ansprüche, worin das heterologe Protein aus der aus Tumornekrosefaktor-a, Interferon-γ, einem Interleukin, einem Zellsuizidprotein und mip-3 bestehenden Gruppe ausgewählt ist.

6. Rekombinanter Adenovirusvektor nach einem der Ansprüche 1 bis 4, worin das heterologe Protein ein Gen zur negativen Selektion ist.

7. Rekombinanter Adenovirusvektor nach Anspruch 6, worin das Gen zur negativen Selektion aus der aus Cytosindesaminase und Thymidinkinase bestehenden Gruppe ausgewählt ist.

8. Verwendung eines rekombinanten Adenovirusvektors nach einem der Ansprüche 1 bis 7 bei der Herstellung eines Medikaments zur Behandlung von Krebs bei einem Säugetier.

9. Verwendung nach Anspruch 8, worin das Medikament der Verabreichung in Verbindung mit einem Chemotherapeutikum oder einem Immunsuppressivum dient.

10. Verfahren zur Herstellung eines rekombinanten Adenovirusvektors zur Verwendung bei der Expression eines heterologen Gens, wobei das Verfahren den Einbau einer aus der aus Pac1, Ca1, Pme1, Swa1, BamH1, BstB1, Ssp1, Nhe1, Stu1 und EcoRV bestehenden Gruppe ausgewählten Restriktionsstelle in die E3-Region des Vektors umfasst;
worin die Restriktionsstellen an relativen Positionen liegen, wie sie in den Fig. 4 bis 7 gezeigt werden;
worin Restriktionsstellen die partielle oder vollständige Deletion eines oder mehrerer in der E3-Region enthaltener Adenovirusgene und Substitution der partiellen oder vollständigen Deletion des einen oder der mehreren Adenovirusgene durch ein heterologes Gen erleichtern; die Restriktionsstelle so positioniert ist, dass das heterologe Gen ein Expressionsmuster aufweist, das sowohl den Zeitablauf als auch das Ausmaß der Expression betreffend ähnlich ist wie jenes des endogenen Adenovirusgens, das es ersetzt, ohne entscheidende Spleiß- und Polyadenylierungssignale zu unterbrechen;
worin allfällige Aminosäureänderungen konservativer Natur sind;
worin die Restriktionsstelle in die Gene der frühen Regionen der E3-Region eingebaut wird, die für das 6,7k- und das gp19k-Protein kodieren; und/oder
worin die Restriktionsstelle in die Gene der frühen Regionen der E3-Region eingebaut wird, die für das 10,4k-, das 14,5k- und das 14,7k-Protein kodieren.

11. Verfahren nach Anspruch 10, weiters umfassend den Schritt des Einführens einer Deletion in eine E1A- oder E1B-Region des Adenovirusvektors, worin die E1 B-Region für ein 55k-Protein kodiert.

12. Verfahren nach Anspruch 10 oder Anspruch 11, das weiters den Schritt des Substituierens eines heterologen Gens anstelle der partiellen oder vollständigen Deletion des einen oder der mehreren Adenovirusgene umfasst, worin das heterologe Gen ein Expressionsmuster aufweist, das sowohl den Zeitablauf als auch das Ausmaß der Expression betreffend ähnlich ist wie jenes des endogenen Adenovirusgens, das es ersetzt.

13. Verfahren nach Anspruch 11, das weiters das Substituieren der Deletionen in der E1b- oder der E1A-Region durch ein Gen umfasst, das für ein heterologes Protein kodiert.

14. Verfahren nach einem der Ansprüche 10 bis 13, worin das heterologe Protein aus der aus Tumornekrosefaktor-a, Interferon-y, einem Interleukin, einem Zellsuizidprotein und mip-3 bestehenden Gruppe ausgewählt ist.

15. Verfahren nach einem der Ansprüche 10 bis 13, worin das heterologe Protein ein Gen zur negativen Selektion ist.

16. Verfahren nach Anspruch 15, worin das Gen zur negativen Selektion aus der aus Cytosindesaminase und Thymidinkinase bestehenden Gruppe ausgewählt ist.

## Revendications

1. Vecteur adénoviral recombinant comprenant des sites de restriction dans la région E3 dudit vecteur qui sont sélectionnés dans le groupe consistant en Pac1, Cla1, Pme1, Swa1, BamH1, BstB1, Ssp1, Nhe1, Stu1 et EcoRV,
dans lequel lesdits sites de restriction ont des positions relatives tel que montré sur les Figures 4 à 7;
dans lequel les sites de restriction sont introduits au moyen d'une mutation, de préférence sans modifier la séquence codante, mais les modifications d'acides aminés sont de nature conservatrice à l'emplacement où elles sont réalisées; et
dans lequel les sites de restriction sont positionnés de sorte à ne pas perturber des signaux d'épissage et de polyadénylation essentiels;
dans lequel les sites de restriction facilitent la délétion partielle ou totale d'un gène ou de gènes adénoviral/adénoviraux contenu(s) dans ladite région E3, et une substitution de ladite délétion partielle ou totale dudit/desdits gène ou gènes adénoviral/adénoviraux par un gène hétérologue,
dans lequel ledit gène hétérologue exhibe un schéma d'expression, à la fois en termes de moment et de degré d'expression, qui est similaire au gène adénoviral endogène qu'il remplace;
dans lequel ledit vecteur comprend des sites de restriction dans les gènes de région précoce de la région E3 qui codent pour les protéines 6.7k et gp19k et/ou
dans lequel ledit vecteur comprend des sites de restriction dans les gènes de région précoce de la région E3 qui codent pour les protéines 10.4k, 14.5k et 14.7k.

2. Vecteur adénoviral recombinant selon la revendication 1, comprenant en outre une délétion dans une région E1A ou E1B dudit vecteur adénoviral, et dans lequel ladite région E1B code pour une protéine 55k.

3. Vecteur adénoviral recombinant selon l'une quelconque des revendications précédentes, comprenant un gène hétérologue qui est substitué à ladite délétion partielle ou totale dudit/desdits gène ou gènes adénoviral/adénoviraux de la région E3, dans lequel ledit gène hétérologue exhibe un schéma d'expression, à la fois en termes de moment et de degré d'expression, qui est similaire au gène adénoviral endogène qu'il remplace.

4. Vecteur adénoviral recombinant selon la revendication 2 ou la revendication 3, dans lequel les délétions de ladite région E1B ou ladite région E1A sont substituées par un gène qui code pour une protéine hétérologue.

5. Vecteur adénoviral recombinant selon l'une quelconque des revendications précédentes, dans lequel ladite protéine hétérologue est sélectionnée dans le groupe consistant en le facteur de nécrose tumorale alpha, l'interféron gamma, une interleukine, une protéine suicide cellulaire et mip-3.

6. Vecteur adénoviral recombinant selon l'une quelconque des revendications 1 à 4, dans lequel la protéine hétérologue est un gène de sélection négative.

7. Vecteur adénoviral recombinant selon la revendication 6, dans lequel le gène de sélection négative est sélectionné dans le groupe consistant en une cytosine désaminase et une thymidine kinase.

8. Utilisation d'un vecteur adénoviral recombinant selon l'une quelconque des revendications 1 à 7, dans la préparation d'un médicament destiné au traitement du cancer chez un mammifère.

9. Utilisation selon la revendication 8, dans laquelle ledit médicament est destiné à être administré en association avec un agent de chimiothérapie ou un agent immunosuppresseur.

10. Procédé pour produire un vecteur adénoviral recombinant destiné à être utilisé pour exprimer un gène hétérologue, ledit procédé consistant à manipuler un site de restriction dans la région E3 dudit vecteur qui est sélectionné dans le groupe consistant en Pac1, Cla1, Pme1, Swa1, BamHl, BstB1, Ssp1, Nhe1, Stu1 et EcoRV,
dans lequel lesdits sites de restriction ont des positions relatives tel que montré sur les Figures 4 à 7;
les sites de restriction facilitant la délétion partielle ou totale d'un gène ou de gènes adénoviral/adénoviraux contenu(s) dans ladite région E3, et une substitution de ladite délétion partielle ou totale dudit/desdits gène ou gènes adénoviral/adénoviraux par un gène hétérologue; ledit site de restriction étant positionné de sorte que ledit gène hétérologue exhibe un schéma d'expression, à la fois en termes de moment et de degré d'expression, qui est similaire au gène adénoviral endogène qu'il remplace sans perturber des signaux d'épissage et de polyadénylation essentiels;
dans lequel de quelconques modifications d'acides aminés sont de nature conservatrice;
dans lequel ledit site de restriction est manipulé dans les gènes de région précoce de la région E3 qui codent pour les protéines 6.7k et gp19k; et/ou
dans lequel ledit site de restriction est manipulé dans les gènes de région précoce de la région E3 qui codent pour les protéines 10.4k, 14.5k et 14.7k.

11. Procédé selon la revendication 10, comprenant en outre l'étape consistant à introduire une délétion dans une région E1A ou E1B dudit vecteur adénoviral, et dans lequel ladite région E1B code pour une protéine 55k.

12. Procédé selon la revendication 10 ou la revendication 11, comprenant en outre l'étape consistant à substituer un gène hétérologue à ladite délétion partielle ou totale dudit/desdits gène ou gènes adénoviral/adénoviraux, dans lequel ledit gène hétérologue exhibe un schéma d'expression, à la fois en termes de moment et de degré d'expression, qui est similaire au gène adénoviral endogène qu'il remplace.

13. Procédé selon la revendication 11, consistant en outre à substituer les délétions de ladite région E1B ou ladite région E1A par un gène qui code pour une protéine hétérologue.

14. Procédé selon l'une quelconque des revendications 10 à 13, dans lequel ladite protéine hétérologue est sélectionnée dans le groupe consistant en le facteur de nécrose tumorale alpha, l'interféron gamma, une interleukine, une protéine suicide cellulaire et mip-3.

15. Procédé selon l'une quelconque des revendications 10 à 13, dans lequel la protéine hétérologue est un gène de sélection négative.

16. Procédé selon la revendication 15, dans lequel le gène de sélection négative est sélectionné dans le groupe consistant en une cytosine désaminase et une thymidine kinase.
